# EUROPEAN PATENT SPECIFICATION

(11) **EP 4 395 754 B1**
(45) Date of publication and mention of the grant of the patent: **11.11.2026**
(21) Application number: 22772930.8
(22) Date of filing: 02.09.2022
(51) Int. Cl.: A61K 31/167, A61K 33/14, A61P 25/00

(54) **METHODS FOR TREATING AUTISM SPECTRUM DISORDERS**
VERFAHREN ZUR BEHANDLUNG VON AUTISMUSSPEKTRUMSTÖRUNG
PROCÉDÉS DE TRAITEMENT DE TROUBLES DU SPECTRE AUTISTIQUE

(30) Priority: 03.09.2021 EP 21194699
(43) Date of publication of application: 10.07.2024
(73) Proprietor: Institut National de la Santé et de la Recherche Médicale (INSERM), 75013 Paris (FR); Centre National de la Recherche Scientifique (CNRS), 75016 Paris (FR); Université de Tours, 37020 Tours Cedex 1 (FR)
(72) Inventor: BECKER, Jérôme, 37200 Tours (FR); LE MERRER, Julie, 37200 Tours (FR)
(74) Representative: Plasseraud IP
(86) International application number: PCT/EP2022/074419
(87) International publication number: WO 2023/031379

(56) References cited:
- US-A1- 2021 106 613
- BECKER JEROME A J ET AL: "Facilitating mGluR4 activity reverses the long-term deleterious consequences of chronic morphine exposure in male mice", NEUROPSYCHOPHARMACOLOGY, SPRINGER INTERNATIONAL PUBLISHING, CHAM, vol. 46, no. 7, 21 December 2020 (2020-12-21), pages 1373 - 1385, XP037458033, ISSN: 0893-133X, [retrieved on 20201221], DOI: 10.1038/S41386-020-00927-X

## Description

### FIELD OF THE INVENTION:

The present invention is in the field of medicine, in particular neurology.

### BACKGROUND OF THE INVENTION:

Autism Spectrum Disorders (ASD) are neurodevelopmental diseases with high heterogeneity and heritability. Their diagnostic is reached in presence of impaired social communication and interaction together with a restricted, repetitive repertoire of behaviors, interests and activities (1). Alongside core symptoms, ASD are often associated with neurobehavioral comorbidities, such as high anxiety, cognitive and motor deficits or epilepsy (2-5). Despite the identification of vulnerability genes and environmental risk factors (6-8), the etiology of ASD remains essentially unknown, making the development of pharmacological treatments for these pathologies a true challenge.

Excitation/inhibition (E/I) imbalance appears as a common mechanistic feature in ASD (*9*, *10*). The heuristic hypothesis of excessive E/I ratio in ASD was initially formulated by Rubenstein and Merzenich (*11*) and raised significant interest as accounting well for reduced GABAergic signaling (*12*, *13*) and high prevalence of epilepsy (10-30%) (*3*) in these pathologies. Indeed, epilepsy is one of the most frequent comorbid medical condition in autism (*5, 14*) and the prevalence of epileptiform EEG or altered resting-state is even higher (*15, 16*), suggesting shared risk factors and/or pathophysiological mechanisms between these pathologies (*17*, *18*). Since its initial formulation, however, the excessive E/I hypothesis in ASD has been challenged by studies in animal models showing instead decreased excitation, which led to a more general concept of altered E/I homeostasis (*10, 19*)*.*

Compromised E/I balance in ASD may result from several neuropathological mechanisms. On the excitation side, glutamatergic transmission was found altered both in patients and animal models, although in different directions depending on genetic mutations/models (*9*, *20, 21*). On the inhibition side, and consistent with impaired GABAergic signaling, decreased levels of GABA (*22*) and expression of GABA_{A} and GABA_{B} (*23, 24*) receptors as well as genetic polymorphisms in GABA_{A} receptor subunits (*25, 26*) have been detected in patients with autism. Accordingly, decreased GABAergic neurotransmission has been reported in several ASD models (*27-31*). Moreover, preclinical studies showed that low doses of benzodiazepines, behaving as positive allosteric modulators (PAMs) of the GABA_{A} receptor (*31, 32*), or the GABA_{B} receptor agonist arbaclofen improve autistic-like behaviors in animal models (*33, 34*). Becker, Jerome AJ et al. report that chronic administration of the mGluR4 positive allosteric modulator (PAM) VU0155041 rescued social behavior, normalized stereotypies and anxiety and blunted locomotor sensitization in morphine abstinent mice (Becker, Jerome AJ, et al. "Facilitating mGluR4 activity reverses the long-term deleterious consequences of chronic morphine exposure in male mice." Neuropsychopharmacology 46.7 (2021): 1373-1385).

Disappointingly, however, clinical trials failed to evidence significant beneficial effects of such compounds in Fragile X syndrome (*35, 36*). Alternatively, it was proposed that GABA neurons remain immature in ASD, failing to shift from high to low intracellular concentrations of chloride ion (Cl⁻), resulting in maintained depolarizing Cl⁻ efflux through activated GABA_{A} receptor (*37*). Intracellular Cl⁻ concentration is under the control of the main Cl⁻ importer NKCC1 (Na⁺-K⁺-2Cl⁻ cotransporter) and the main chloride exporter KCC2. Therefore blocking NKCC1 using the loop diuretic and antiepileptic drug (*38, 39*) bumetanide appeared a promising therapeutic approach in ASD. Accordingly, bumetanide was found to improve autistic-like phenotype in rodent models of ASD (*40*) and to relieve autistic behavior in small cohorts of patients (*41*, *42*). Clinical benefit, however, failed to be confirmed in a larger clinical trial, except for a reduction of repetitive behavior (*43*).

Bromide ion (Br⁻) was the first effective treatment identified for epilepsy (*44*), long used also as an anxiolytic and hypnotic medication (45). With the advent of novel antiepileptic and anxiolytic drugs, more specific and supposedly less toxic, the use of Br⁻ was progressively dropped down, although it remains a valuable tool to treat refractory seizures (*46*, *47*). As regards its mechanism of action, Br⁻ shares similar chemical and physical similarities with Cl⁻ allowing it substituting Cl⁻ in multiple cellular mechanisms. These include anion efflux through activated GABA_{A} receptor, with higher permeability to Br⁻ compared to Cl⁻ resulting in neuronal hyperpolarization (48). Br⁻ can also replace Cl⁻ in mechanisms where the NKCC and KCC cotransporters are involved (*49, 50*). In view of the E/I imbalance theory, these properties point to Br⁻ as an interesting candidate for ASD treatment (WO2018/096184).

### SUMMARY OF THE INVENTION:

The present invention is defined by the claims. The references to methods of treatment by therapy of this description are to be interpreted as references to compounds, pharmaceutical compositions and medicaments of the present invention for use in those methods

In particular, the present invention relates to a method of treating an Autism Spectrum Disorder (ASD) in a subject in need thereof comprising administering to the subject a therapeutically effective combination comprising a bromide salt and a Positive Allosteric Modulator (PAM) of mGlu4 receptor.

### DETAILED DESCRIPTION OF THE INVENTION:

In the present study, the Inventors assessed the effects of chronic sodium bromide administration on core autistic-like symptoms: social deficit and stereotypies, as well as on a frequent comorbid symptom: anxiety, in three genetic mouse models of autism: *Oprm1*^{*-*/}*⁻, Fmr1*^{*-* /*-*} and *Shank3*^{*Δex13-16-*/*-*} mice by means of thorough behavioral assessment. Altered E/I balance and/or modified expression of genes involved in this balance have been reported for these three models (*30, 51-55*); the *Oprm1* knockout model presents the advantage of limited impact on learning performance (52), allowing better disentangling autistic features from cognitive deficit. They evidenced that Br⁻ treatment alleviates most of the behavioral deficits observed in these mice, and increases expression of various genes within the social brain circuit. They unraveled that Br- not only increases mGlu4 receptor gene expression but also potentiates the effects of the mGlu4 PAM VU0155041 as well as its agonist glutamate, in *Oprm1*^{*-*/*-*} mice and in heterologous cells. Their data reveal the therapeutic potential of Br⁻ administration and its combination with a positive allosteric modulator (PAM) of mGlu4 receptor for the treatment of ASD.

### Method for treating Autism Spectrum Disorders

**In a first aspect,** the present invention relates to a method of treating an Autism Spectrum Disorder (ASD) in a subject in need thereof comprising administering to the subject a therapeutically effective combination comprising a bromide salt and a Positive Allosteric Modulator (PAM) of mGlu4 receptor.

As used herein, the term **"subject"** or **"patient"** denotes a mammal, preferably a human. Typically, a subject according to the invention refers to any subject afflicted with or susceptible to be afflicted with Autism Spectrum Disorders (ASD).

As used herein, the term **"Autism Spectrum Disorders"** or **"ASD"** denotes a developmental disorder that affects communication and behaviour in a subject. According to the Diagnostic and Statistical Manual of Mental Disorders, autism is characterized by difficulty with communication and interaction with others, restricted interests and activities and repetitive behaviour (American Psychiatric Association. (2013). Diagnostic and statistical manual of mental disorders (5th ed.). Arlington, VA: Author).

As used herein, the term **"treatment"** or **"treat"** refer to both prophylactic or preventive treatment as well as curative or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By **"therapeutic regimen"** is meant the pattern of treatment of an illness, e.g., the pattern of dosing used during therapy. A therapeutic regimen may include an induction regimen and a maintenance regimen. The phrase **"induction regimen"** or **"induction period"** refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a **"loading regimen",** which may include administering a greater dose of the drug than a physician would employ during a maintenance regimen, administering a drug more frequently than a physician would administer the drug during a maintenance regimen, or both. The phrase **"maintenance regimen"** or **"maintenance period"** refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at a regular interval, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

In particular, the method of the present invention is particularly useful to alleviate at least one symptom of ASD in a subject in need thereof, wherein the at least one symptom is social behaviour deficits, stereotypic behaviours and/or excessive anxiety.

As used herein, the term "social **behaviour deficits"** denotes a state when a subject suffers from difficulties with verbal and non-verbal communication. As example, symptoms may include unusual or inappropriate body language, gestures and facial expressions, lack of interest in other people or in sharing interests or achievements, unlikely to approach others or to pursue social interactions, comes across as aloof and detaches, prefers to be alone, difficulties to understand other people's feelings, reactions and nonverbal cues, resistance to being touched, difficulties or failure to make friends, delay in learning how to speak or does not talk at all, speaking in an atypical tone of voice or with an odd rhythm or pitch, repeating words or phrases over and over without communicative intent, trouble starting a conversation of keeping it going, difficulties communicating needs or desires, does not understand simple statements or questions and/or taking what is said literally, missing humour, irony and sarcasm (American Psychiatric Association. (2013). Diagnostic and statistical manual of mental disorders (5th ed.). Arlington, VA: Author).

As used herein, the term **"stereotypic behaviours"** denotes a state when a subject suffers from restricted, rigid and/or obsessive in their behaviours, activities and/or interests. As example, symptoms may include cognitive deficit, repetitive body movements, moving constantly, obsessive attachment to unusual objects, preoccupation with a narrow topic of interests sometimes involving numbers or symbols, strong need for sameness, order and routines (gets upset by change in their routine or environment), clumsiness, atypical posture or odd ways of moving, fascinated by spinning objects, moving pieces or parts of toys and/or hyper or hypo reactive to sensory input, (American Psychiatric Association. (2013). Diagnostic and statistical manual of mental disorders (5th ed.). Arlington, VA: Author).

As used herein, the term **"excessive anxiety"** denotes a state when a subject suffers from a frequent, intense, excessive and persistent worry and fear about life circumstances. As example, symptoms may include having difficulty concentrating or sleeping, being irritable, having muscle tension, difficulty controlling feelings or worry, dizziness or heart palpitations, feeling restless or worried (U.S. Department of Health and Human Services, National Institutes of Health, National Institute of Mental Health. (2015). NIMH Strategic Plan for Research (NIH Publication No. 02-2650). Retrieved from http://www.nimh.nih.gov/about/strategic-planning-reports/index.shtml).

As used herein, the term **"bromide salt"** has its general meaning in the art and refers to an inorganic compound consisting of an ionic assembly of brome in a cationic form and one anion. In some embodiments, the bromide salt is selected from potassium, sodium, ammonium, calcium or lithium salts, alone or mixtures of 2, 3, 4 or 5 of said salts.

In a particular embodiment, the bromide salt is sodium bromide (NaBr). In another particular embodiment, the bromide salt is potassium bromide (KBr).

As used herein, the term **"Positive Allosteric Modulator"** or **"PAM"** denotes a group of substance that bind to a receptor to increase agonist affinity (i.e. increasing the probability that an agonist will bind to the receptor) and/or efficacy (i.e. increasing its ability to activate the receptor) (Abdel-Magid AF. Allosteric modulators: an emerging concept in drug discovery. ACS Med Chem Lett. 2015;6(2):104-107. Published 2015 Jan 8. doi:10. 1021/m15005365*).*

As used herein, the term **"mGlu4 receptor"** or **"Metabotropic Glutamate Receptor 4"** denotes a protein belonging to group III of the metabotropic glutamate receptor family. The metabotropic glutamate receptors are a family of G protein-coupled receptors, that have been divided into 3 groups on the basis of sequence homology, putative signal transduction mechanisms, and pharmacologic properties. Group III receptors are linked to the inhibition of the cyclic AMP cascade. Ligand binding causes a conformation change that triggers signaling via guanine nucleotide-binding proteins (G proteins) and modulates the activity of down-stream effectors. Signalling inhibits adenylate cyclase activity (Wu S, Wright RA, Rockey PK, et al. Group III human metabotropic glutamate receptors 4, 7 and 8: molecular cloning, functional expression, and comparison of pharmacological properties in RGT cells. Brain Res Mol Brain Res. 1998;53(1-2):88-97. doi:10.1016/s0169-328(97)00277-5).

MGlu4 receptor is encoded by the *GRM4* gene (Gene ID: 2914; Ensembl: ENSG00000124493; OMIM: 604100; UniProt: Q14833).

As example, a PAM of mGlu4 receptor may be VU0155041 (CAS number: 1093757-42-6), PXT-002331 (CAS number: 2133294-96-7), DT-1687 (CAS number: 1883329-53-0), VU0361737 (CAS number: 1161205-04-4), VU0364770 (CAS number: 61350-00-3), VU0418506, VU001171, VU0652957 (CAS number: 1976050-09-5), PHCCC (CAS number: 179068-02-1*)* or AB120043 (CAS number: 68-19-9).

In a particular embodiment, the positive allosteric modulator of mGlu4 receptor is VU0155041, having the formula (I):

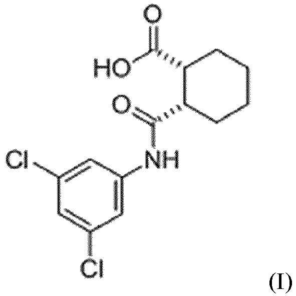

As used herein, the term **"combination"** is intended to refer to all forms of administration that provide a first drug together with a further (second, third...) drug. The drugs may be administered simultaneous, separate or sequential and in any order. Drugs administered in combination have biological activity in the subject to which the drugs are delivered. Within the context of the invention, a combination thus comprises at least two different drugs, and wherein one drug is the bromide salt and wherein the other drug is the positive allosteric modulator of mGlu4 receptor. According to the present invention, the combination of the present invention results in a synergistic effect. As used herein, the term **"synergistic effect"** or grammatical variations thereof means and includes a cooperative action encountered in a combination of two or more active compounds in which the combined activity of the two or more active compounds exceeds the sum of the activity of each active compound alone.

In some embodiments, the bromide salt is administrated chronically. As used herein, the term **"chronically"** means in a persistent and recurring way, but not necessarily at regular intervals.

In some embodiments, the bromide salt is administrated at least once per day. In some embodiments, the bromide salt is administrated at least once per week. In some embodiments, the bromide salt is administrated at least once per two weeks.

In some embodiments, the Positive Allosteric Modulator (PAM) of mGlu4 is administrated at least once per day. In some embodiments, the Positive Allosteric Modulator (PAM) of mGlu4 is administrated at least once per week. In some embodiments, Positive Allosteric Modulator (PAM) of mGlu4 is administrated at least once per two weeks.

A used herein, the term **"therapeutically effective amount"** above described is meant a sufficient amount of the bromide salt and the Positive Allosteric Modulator (PAM) of mGlu4 receptor compounds for achieving a therapeutic effect (alleviate at least one symptom of ASD). It will be understood, however, that the total daily usage of the compounds and compositions of the present invention will be decided by the attending physician within the scope of sound medical judgment. The specific therapeutically effective dose level for any particular subject will depend upon a variety of factors including the disorder being treated and the severity of the disorder; activity of the specific compound employed; the specific composition employed, the age, body weight, general health, sex and diet of the subject; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved.

In some embodiments, the bromide salt is administrated at least 10 mg/kg.

In some embodiments, the bromide salt is administrated at least 30 mg/kg.

In some embodiments, the bromide salt is administrated at least 70 mg/kg.

In some embodiments, the bromide salt is administrated at least 125 mg/kg.

In some embodiments, the bromide salt is administrated at least 145 mg/kg.

In some embodiments, the bromide salt is administrated at least 250 mg/kg.

In some embodiments, the bromide salt is administrated at least 500 mg/kg.

In some embodiments, the allosteric modulator of mGlu4 receptor is administrated at least 1 mg/kg.

A further object of the present invention relates to a method for enhancing the potency of a positive Allosteric Modulator (PAM) of mGlu4 receptor administered to a subject suffering from an Autism Spectrum Disorder (ASD), the method comprising administering to the subject a pharmaceutically effective amount of a positive Allosteric Modulator (PAM) of mGlu4 receptor in combination with a bromide salt.

In some embodiments, the present invention relates to a method for enhancing the potency of VU0155041 administered to a subject suffering from an Autism Spectrum Disorder (ASD), the method comprising administering to the subject a pharmaceutically effective amount of VU0155041 in combination with sodium bromide.

### Therapeutic composition

**In a third aspect,** the present invention relates to a therapeutic composition comprising a bromide salt and a Positive Allosteric Modulator (PAM) of mGlu4 receptor for use in the treatment of Autism Spectrum Disorders (ASD) in a subject in need thereof.

In some embodiments, the present invention relates to a therapeutic composition comprising sodium bromide and VU0155041 for use in the treatment of Autism Spectrum Disorders (ASD) in a subject in need thereof.

Typically, the bromide salt and the Positive Allosteric Modulator (PAM) of mGlu4 receptor may be combined with pharmaceutically acceptable excipients, and optionally sustained-release matrices, such as biodegradable polymers, to form therapeutic compositions.

**"Pharmaceutically" or "pharmaceutically acceptable"** refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Galenic adaptations may be done for specific delivery in the small intestine or colon. Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising the bromide salt and the Positive Allosteric Modulator (PAM) of mGlu4 receptor of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The bromide salt and the Positive Allosteric Modulator (PAM) of mGlu4 receptor of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifusoluble agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active polypeptides in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. Multiple doses can also be administered. In addition to the bromide salt and the Positive Allosteric Modulator (PAM) of mGlu4 receptor of the invention formulated for parenteral administration, such as intravenous or intramuscular injection, other pharmaceutically acceptable forms include, e.g. tablets or other solids for oral administration; liposomal formulations; time release capsules; and any other form currently used.

In some embodiments, the present invention relates to a therapeutic composition comprising sodium bromide and VU0155041 for use in the treatment of Autism Spectrum Disorders (ASD) in a subject in need thereof.
In some embodiments, the therapeutic composition of the present invention may comprise at least one further therapeutic active agent. As example, the at least one further therapeutic active agent may be a diuretic such as bumetanide (CAS number: 28395-03-1*)*, an anxiolytic such as clobazam (CAS number: 22316-47-8*),* clorazepate (CAS number: 57109-90-7*),* nordazepam *(*CAS number: 1088-11-5*),* diazepam (CAS number: 439-14-5*),* prazepam (CAS number: 2955-38-6), alprazolam (CAS number: 28981-97-7*),* bromazepam (CAS number: 1812-30-2*),* lorazepam (CAS number: 846-49-1*),* oxazepam (CAS number: 604-75-1*),* hydroxyzine (CAS number: 68-88-2*),* an anti-psychotic such as risperidone (CAS number: 106266-06-2*),* aripiprazole (CAS number: 129722-12-9*),* olanzapine (CAS number: 132539-06-1*), a* neuroleptic such as lamotrigin (CAS number: 84057-84-1*),* carbamazepine (CAS number: 298-46-4), valpromid (CAS number: 2430-27-5*),* a muscle relaxant such as baclofen (CAS number: 1134-47-0*),* an anti-depressive such as fluoxetine (CAS number: 54910-89-3*),* sertralin *(*CAS number: 79617-96-2*),* paroxetine (CAS number: 61869-08-7*)* and/or a stimulant such as ritalin *(*CAS number: 113-45-1*)* or caffeine (CAS number: 58-08-2).

In some embodiments, the therapeutic composition of the present invention may comprise at least one further compound such as glutamate, B6 vitamin and/or B12 vitamin.

The invention will be further illustrated by the following figures and examples. However, these examples and figures should not be interpreted in any way as limiting the scope of the present invention.

### FIGURES:

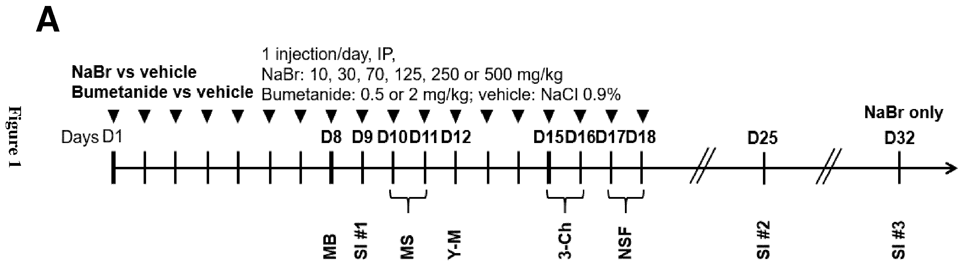

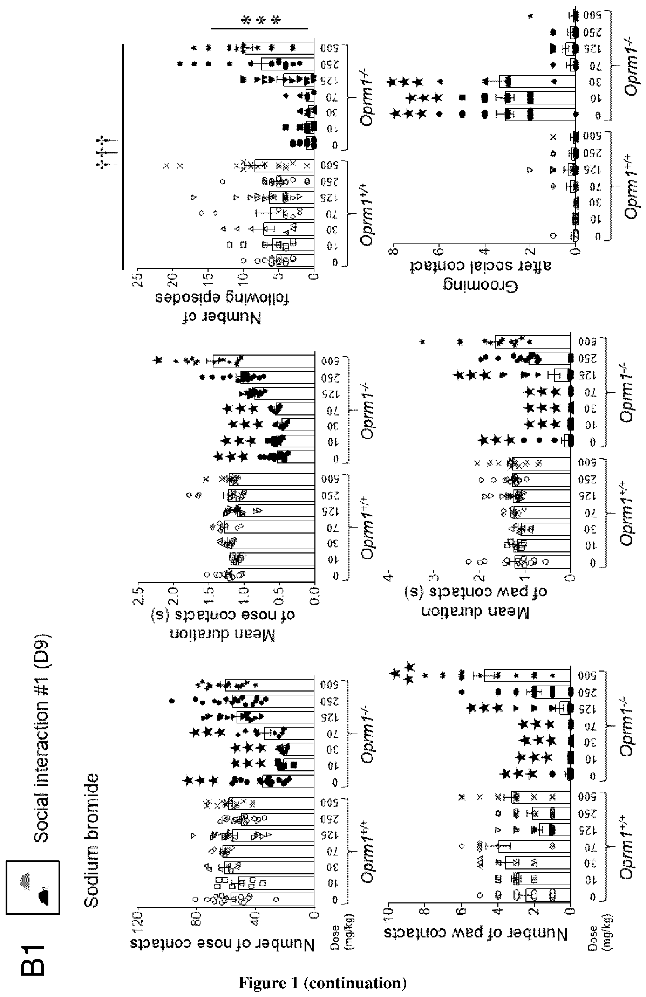

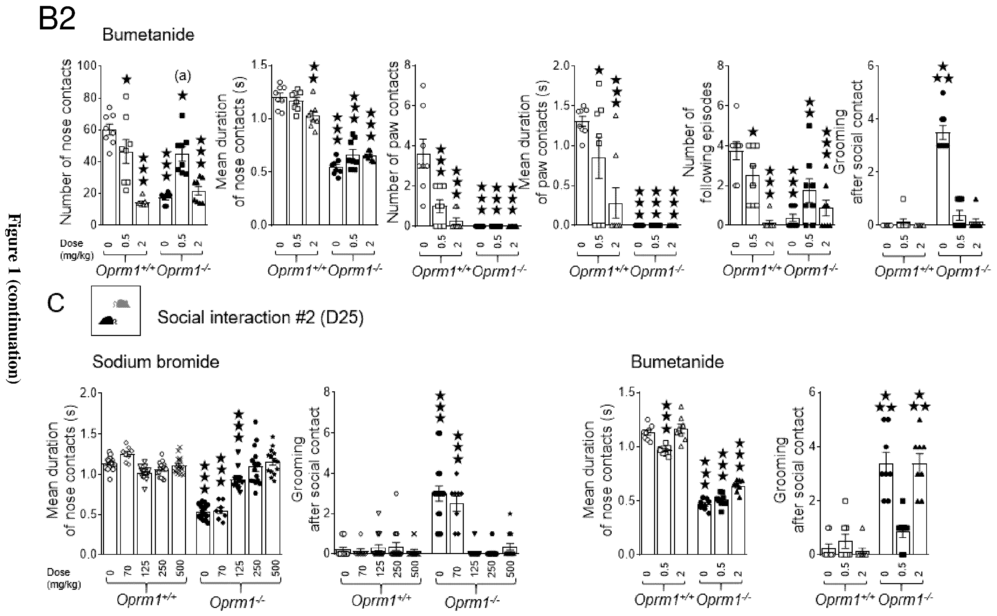

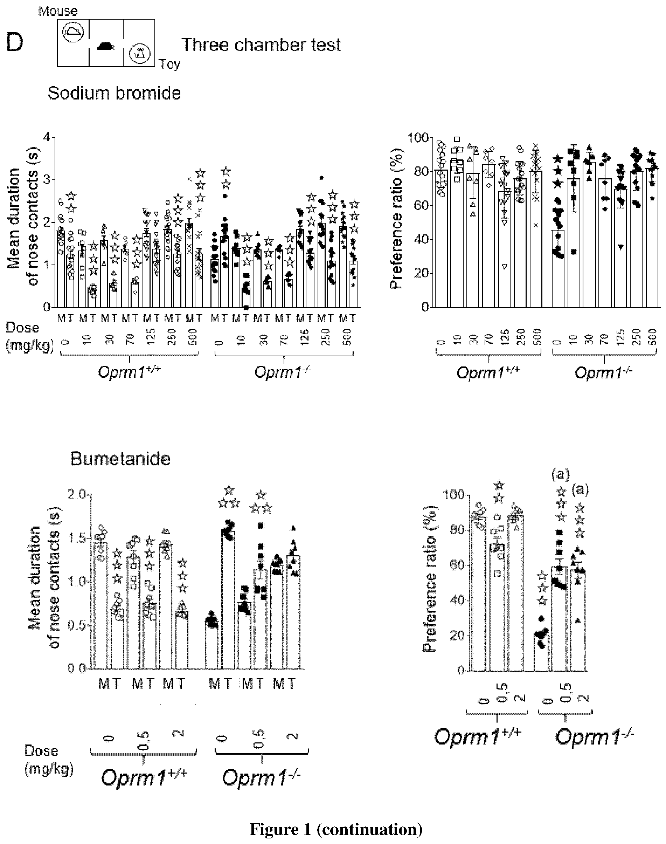

**Figure 1**. **Chronic sodium bromide dose-dependently relieved social behavior deficits in *Oprm1*^{*-*/*-*} mice, demonstrating superior effects to chronic bumetanide. (A)** *Oprm1*^{*+*/*+*} and *Oprm1*^{*-*/*-*} mice were treated either with NaBr (0, 125-500 mg/kg: n=14-20 mice per genotype and dose; 10-70 mg/kg: n=8 mice per genotype and dose) or with bumetanide (0, 0.5 and 2 mg/kg: n=8-10 mice per genotype and dose) once daily for 18 days. Behavioral testing started on D8; social interaction was retested 1 week and 2 weeks after cessation of chronic administration. **(B)** In the direct social interaction test (D9), chronic NaBr **(B1)** administration relieved social deficits of *Oprm1* null mice in a dose-dependent manner for doses over 125 mg/kg; it had no detectable effect in *Oprm1*^{*+*/*+*} mice. Bumetanide **(B2)** had only partial effects, increasing the number of nose contacts (low dose) and suppressing grooming after social contact; at the highest dose, it impaired social interaction in wild-type controls. (C) One week after cessation of treatment, beneficial effects of bromide administration were preserved for doses over 125 mg/kg; effects of bumetanide one duration of nose contacts and grooming after social contact were still detectable. **(D)** In the three-chamber test, NaBr treatment rescued social preference in *Oprm1* mutants since the dose of 10 mg/kg, while bumetanide increased their interest for the mouse without reducing their abnormal interest for the object. Results are shown as scatter plots and mean ± sem. Daggers: genotype effect, asterisks: treatment effect, solid stars: genotype x treatment interaction (comparison to wild-type vehicle condition), open stars: genotype x treatment x stimulus interaction (mouse versus object comparison), (a) genotype x treatment interaction (comparison with knockout vehicle condition, p<0.001) (two-way ANOVA or three-way ANOVA with stimulus as repeated measure, followed by Newman-Keuls post-hoc test). One symbol: p<0.05, two symbols: p<0.01; three symbols: p<0.001. 3-Ch: 3-chamber test, M: mouse, MB: marble burying, MS: motor stereotypies, NSF: novelty-suppressed feeding, SI: social interaction, T: toy, Y-M: Y-maze.

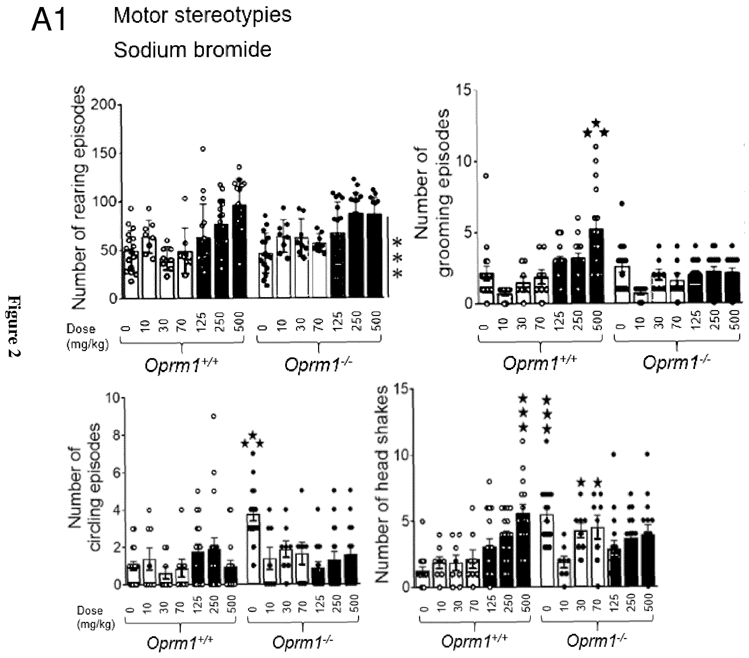

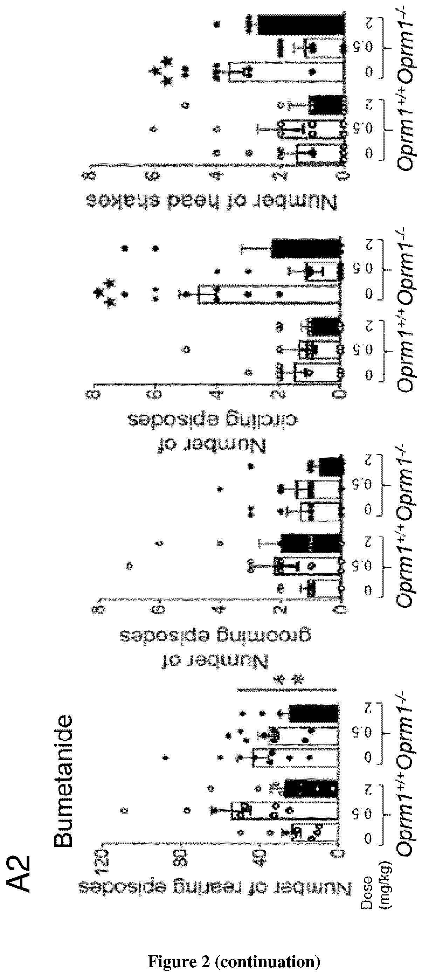

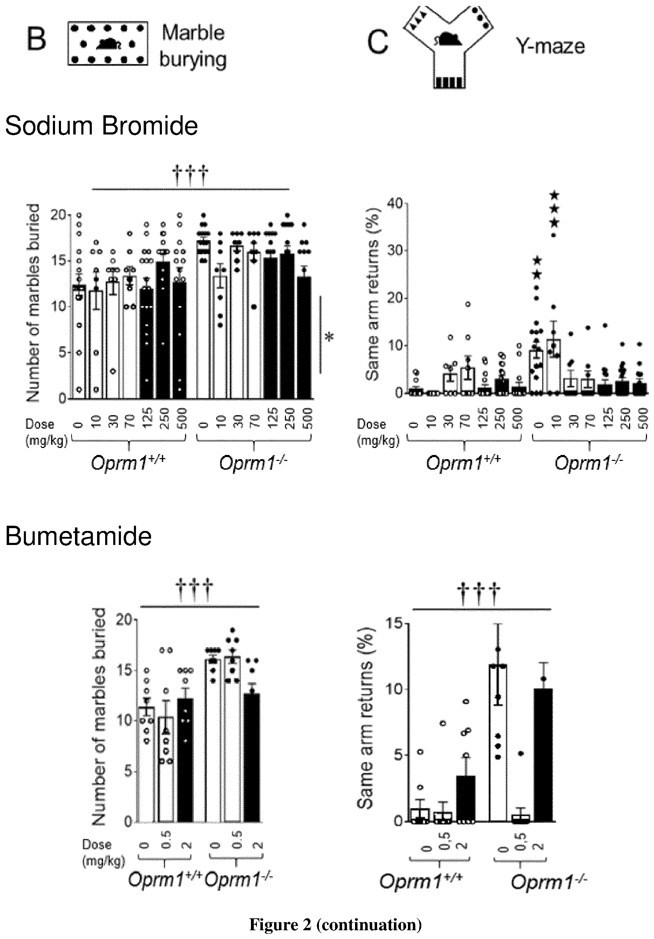

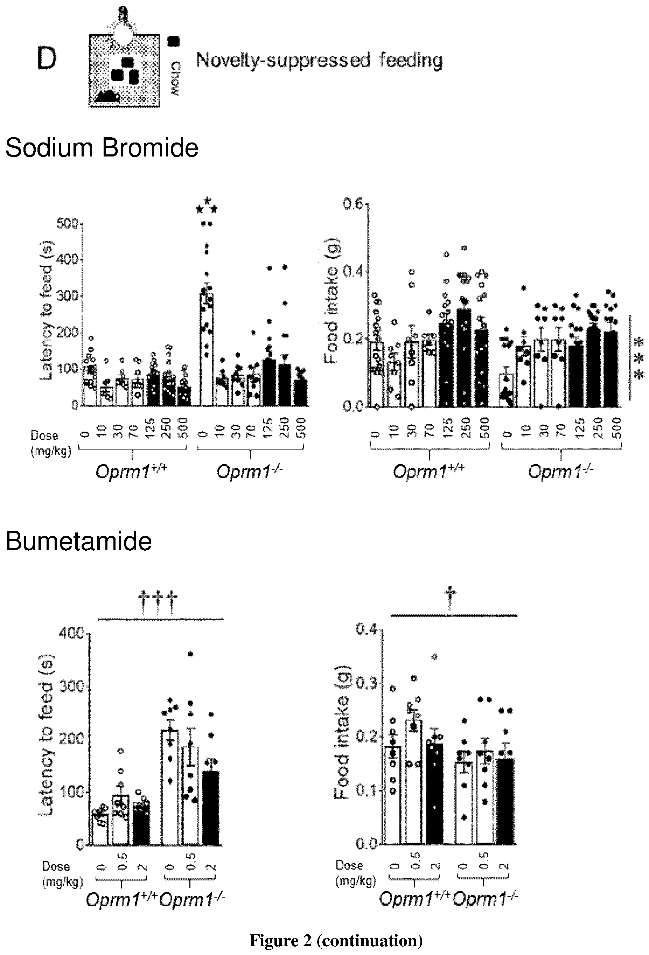

**Figure 2**. **Chronic sodium bromide treatment reduced stereotypic behaviors and anxiety in *Oprm1*^{*-*/*-*} mice.** See timeline of experiments and animal numbers in **Figure 1A****. (A)** Chronic NaBr (**A1**) administration suppressed stereotypic circling episodes in *Oprm1*^{*-*/*-*} mice since the dose of 10 mg/kg and less consistently reduced the number of head shakes (doses over 125 mg/kg). In wild-type controls, NaBr at 500 mg/kg increased the frequency of grooming episodes and head shakes. Bumetanide **(A2)** suppressed stereotypic circling and head shakes. **(B)** In the marble burying test, chronic bromide globally increased the number of buried marbles in both mouse lines; bumetanide failed to demonstrate significant effects. **(C)** In the Y-maze, NaBr suppressed perseverative same arm returns from the dose of 70 mg/kg; bumetanide had not significant effect despite an obvious tendency for the dose of 0.5 mg/kg to relieve perseveration. **(D)** In the novelty-suppressed feeding test, sodium bromide normalized the latency to feed in *Oprm1* null mice to wild-type levels since the lowest dose tested and increased food intake in all mice; bumetanide had no significant effect in this test. Results are shown as scatter plots and mean ± sem. Daggers: genotype effect, asterisks: treatment effect, solid stars: genotype x treatment interaction (comparison with wild-type vehicle condition) (two-way ANOVA followed by Newman-Keuls post-hoc test). One symbol: p<0.05, two symbols: p<0.01; three symbols: p<0.001.

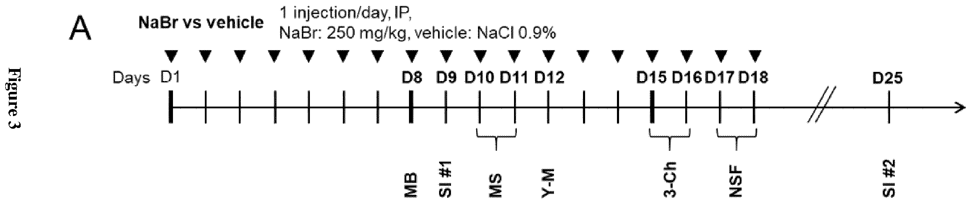

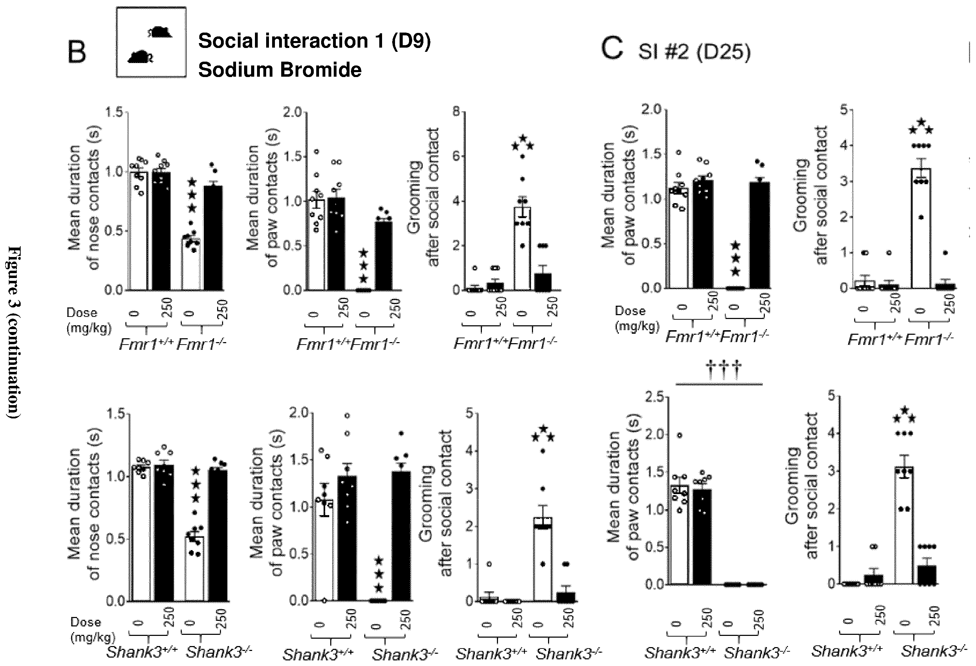

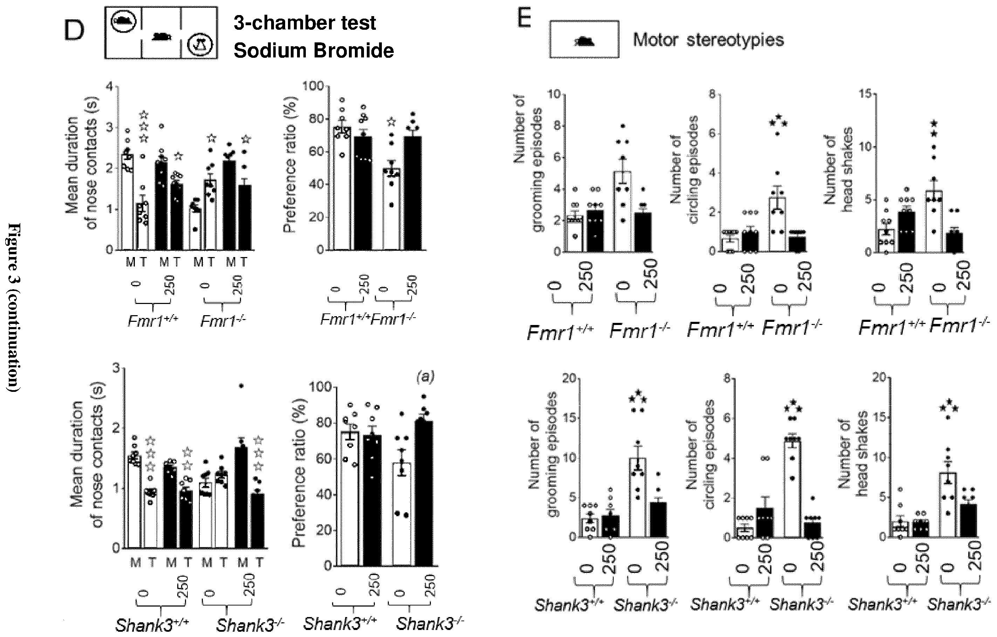

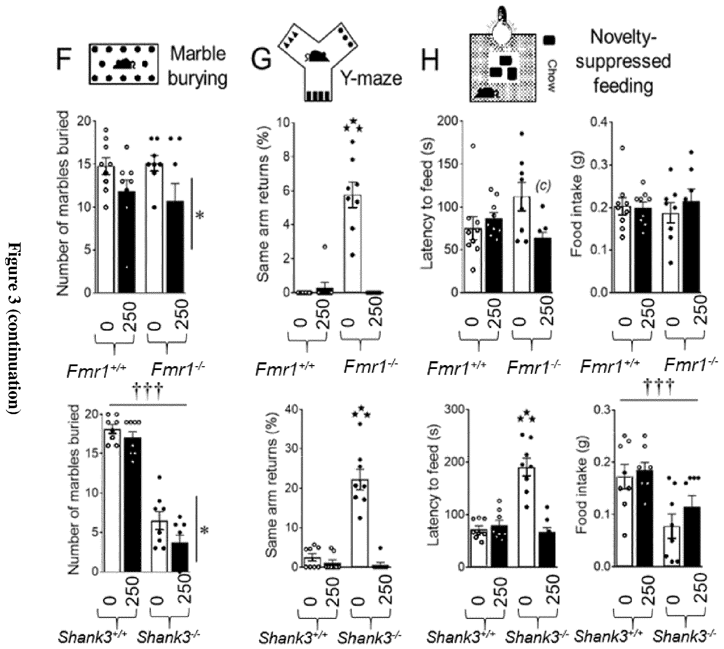

**Figure 3****. Chronic sodium bromide administration relieved social behavior deficits in *Fmr1*^{*-*/*-*} and *Shank3*^{Δ*ex13-16-*/*-*} mice. (A)** *Fmr1*^{*-*/*-*} or *Shank3*^{Δ*ex13-16-*/*-*}("Shank3^{-/-}") and their respective wild-type counterparts were treated with NaBr (0 or 250 mg/kg; n=8 mice per genotype and treatment) once daily for 18 days. Behavioral testing started on D8; social interaction was retested 1 week (D25) after cessation of chronic administration. **(B)** In the direct social interaction test, chronic NaBr treatment normalized interaction parameters to wild-type levels in both *Fmr1* and *Shank3* mutant lines. **(C)** One week after cessation of treatment, these beneficial effects were fully maintained in *Fmr1*^{*-*/*-*} mice while they were detected for some parameters only in *Shank3*^{Δ*ex13-16-*/*-*} mice. **(D)** In the 3-chamber test, chronic NaBr administration rescued preference for making longer nose contacts with the mouse in *Shank3*^{Δ*ex13-16-*/*-*} mice, resulting in increased preference ratio. **(E)** Chronic sodium bromide treatment suppressed stereotypic circling and head shakes in *Fmr1*^{*-*/*-*} and *Shank3*^{Δ*ex13-16-*/*-*} mice and normalized grooming in the latter. **(F)** NaBr reduced marble burying in *Fmr1*^{*-*/*-*} and *Fmr1*^{*+*/*+*} while it had no effect on reduced burying in *Shank3*^{Δ*ex13-16-*/*-*} mice. **(G)** During Y-maze exploration, chronic NaBr suppressed perseverative same arm returns in both *Fmr1* and *Shank3* mutant lines. **(H)** Finally, in the novelty-suppressed feeding test, sodium bromide-treated *Fmr1*^{*-* /*-*} or *Shank3*^{Δ*ex13-16-*/*-*} mice displayed reduced or normalized latency to feed, respectively, but no modification in their food intake. Results are shown as scatter plots and mean ± sem. Daggers: genotype effect, solid stars: genotype x treatment interaction (comparison to wild-type vehicle condition), open stars: genotype x treatment x stimulus interaction (mouse versus object comparison), (a) genotype x treatment interaction (comparison with knockout vehicle condition, p<0.001), (c) genotype x treatment interaction (comparison to knockout vehicle condition, p<0.05) (two-way ANOVA or three-way ANOVA with stimulus as repeated measure, followed by Newman-Keuls post-hoc test). One symbol: p<0.05, two symbols: p<0.01; three symbols: p<0.001. 3-Ch: 3-chamber test, AAR: alternate arm returns, M: mouse, MB: marble burying, MS: motor stereotypies, NSF: novelty-suppressed feeding, SAR: same arm returns, SPA: spontaneous alternation, SI: social interaction, T: toy, Y-M: Y-maze.

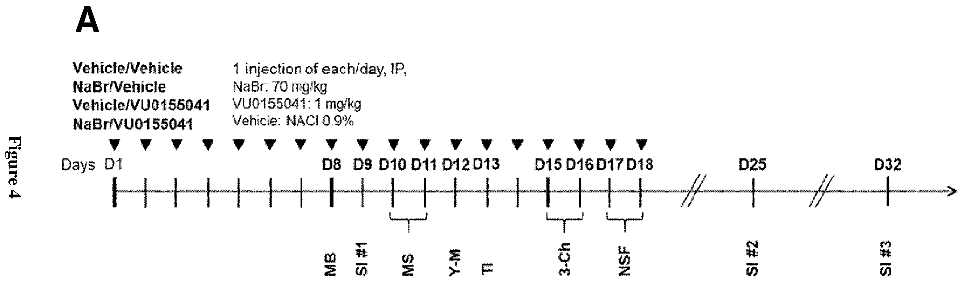

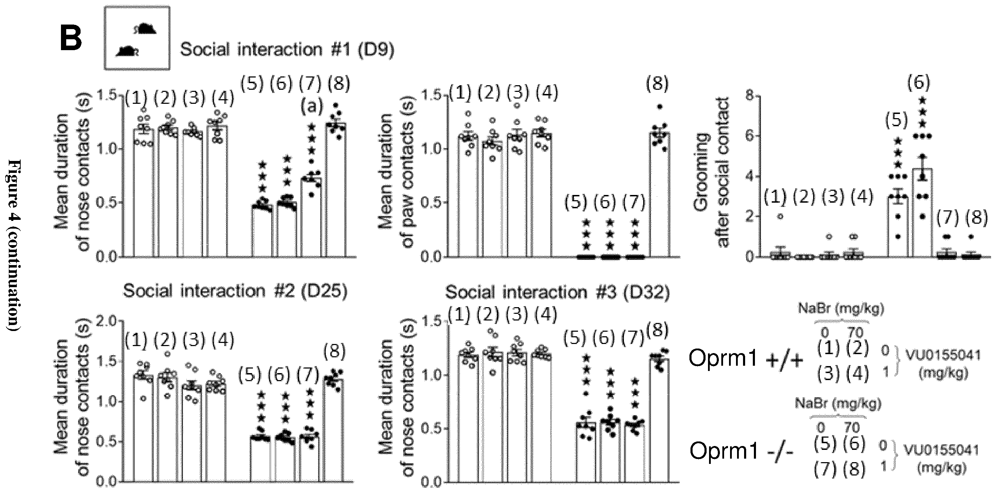

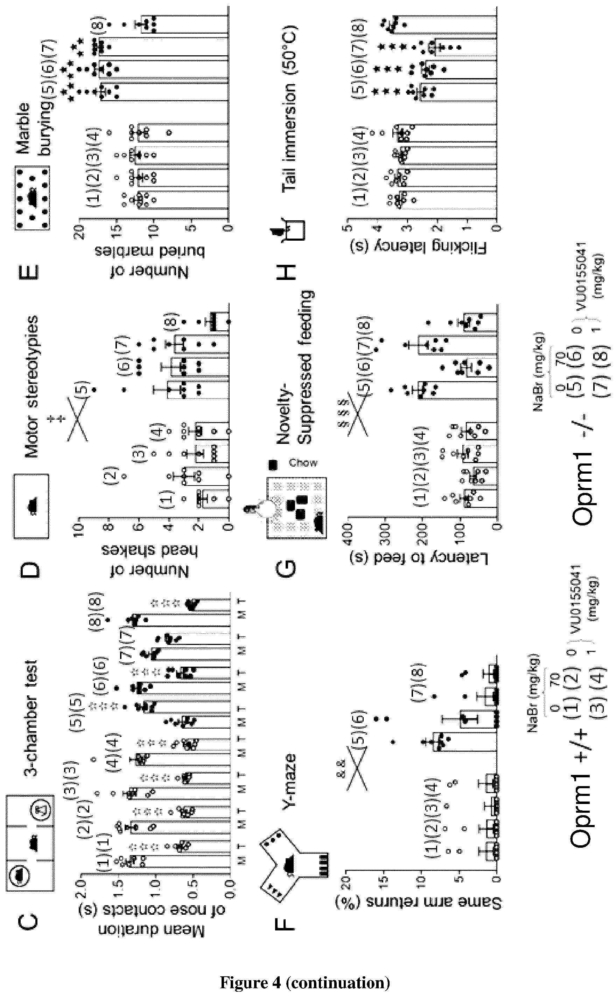

**Figure 4****. Beneficial effects of sodium bromide and VU0155041, a positive allosteric modulator of mGlu4 receptors, were synergistic in *Oprm1*^{*-*/*-*} mice. (A)** *Oprm1*^{*+*/*+*} and *Oprm1*^{*-* /*-*} mice were treated either with vehicle, NaBr (70 mg/kg), VU0155041 (1 mg/kg) or NaBr and VU0155041 (70 and 1 mg/k, respectively; 8 mice per genotype and dose) once daily for 18 days. Behavioral testing started on D8; social interaction was retested 1 week and 2 weeks after cessation of chronic administration. **(B)** In the direct social interaction test, NaBr and VU0155041 treatments demonstrated synergistic effects in restoring the duration of nose and paw contacts in *Oprm1*^{*-*/*-*} mice. VU0155041 at 1 mg/kg, however, was sufficient to suppress grooming after social contact. Beneficial effects of combined NaBr/VU0155041 were fully maintained one and two weeks after cessation of treatment. **(C)** In the 3-chamber test, VU0155041 at 1 mg/kg increased the duration of nose contacts with the mouse to that of the toy in *Oprm1*^{*-*/*-*} mice; NaBr at 70 mg/kg and combined NaBr/VU0155041 treatment fully restored longer nose contacts with the mouse. **(D)** Combined NaBr/VU0155041 administration reduced head shakes in *Oprm1*^{*-*/*-*} and *Oprm1*^{*+*/*+*} mice and **(E)** normalized marble burying in *Oprm1* null mice only. **(F)** VU0155041 treatment was sufficient to suppress perseverative same arm returns in *Oprm1*^{*-*/*-*} mice exploring the Y-maze and **(G)** NaBr administration was sufficient to normalize their latency to feed in novelty-suppressed feeding test. **(H)** In the tail immersion test at 50°C, only combined NaBr/VU0155041 treatment restored flicking latency of *Oprm1*^{*-*/*-*} mice to wild-type levels. Results are shown as scatter plots and mean ± sem. Solid stars: genotype x NaBr x VU0155041 interaction (comparison to wild-type vehicle condition), open stars: genotype x stimulus x NaBr x VU0155041 interaction (mouse versus object comparison), (a) genotype x NaBr x VU0155041 interaction (comparison with knockout vehicle condition, p<0.001), double dagger: NaBr x VU0155041 interaction, ampersand: genotype x VU0155041 interaction, section: genotype x NaBr interaction (three-way or four-way ANOVA followed by Newman-Keuls post-hoc test). One symbol: p<0.05, two symbols: p<0.01; three symbols: p<0.001. 3-Ch: 3-chamber test, M: mouse, MB: marble burying, MS: motor stereotypies, NSF: novelty-suppressed feeding, SI: social interaction, T: toy, TI: tail immersion, Y-M: Y-maze.

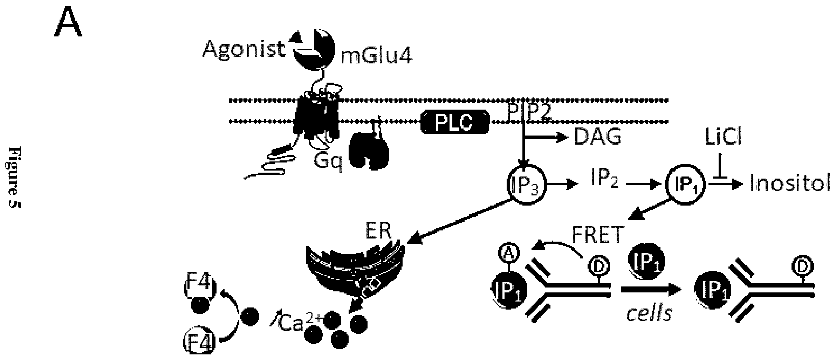

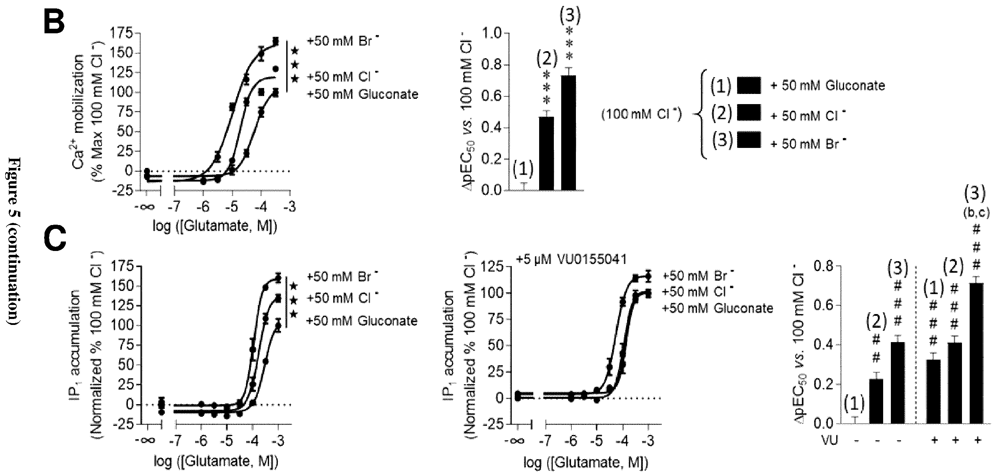

**Figure 5****. Bromide ions behave as PAMs of the mGlu4 receptor and shows synergistic effects with the mGlu4 PAM VU0155041. (A)** Signaling cascade of mGlu4 receptor when coupled with the chimeric G-protein Gα_{qi9} (to allow the recruitment of the phosphoinositide pathway) and experimental principles of the calcium mobilization (Panel B) and IP1 accumulation assays (Panel C). **(B)** In the calcium mobilization assay, bromide ions behave as PAM of mGlu4, demonstrating broader effects than chloride ions on both pEC₅₀ and Eₘₐₓ. **(C)** In the IP1 accumulation assay, bromide confirmed its PAM effects; when supplemented with VU0155041, facilitation of mGlu4 signaling was even increased, as seen by a further rise in ΔpEC50. Results are shown as mean ± SEM of three independent experiments realized in triplicates. Solid stars: effect of ion concentration on Eₘₐₓ; asterisks: effect of ion concentration on ΔpEC₅₀, comparison with physiological conditions (100 mM Cl⁻ + 50 mM gluconate); (a): comparison with high chloride condition (150 mM Cl⁻, p<0.0001); hashtags: effect of ion concentration and VU0155041 on ΔpEC₅₀, comparison with physiological conditions (100 mM Cl⁻ + 50 mM gluconate); (b) and (c): comparison with bromide conditions (100 mM Cl⁻ + 50 mM Br-) with or without VU0155041 (p<0.0001) (One-way ANOVA, followed by Tukey's post-hoc test). Two symbols: p<0.001, three symbols: p<0.0001. DAG: diacylglycerol; ER: endoplasmic reticulum; F4: Fluo4 calcium probe; FRET: fluorescence resonance energy transfer; PLC: phospholipase C; IP_{1/2/3}: inositol mono/di/triphosphate.

### EXAMPLE:

### MATERIAL AND METHODS

### Animals, breeding procedures and housing conditions

The *Oprm1*^{*-*/*-*} (B6.129S2-Oprm1^{tm1Kff}/J) (58) and *Shank3*^{Δ*ex13-16-*/*-*} (B6.129-Shank3^{*tm2Gfng*/*J*}, so called *Shank3B*^{*-*/}*⁻,* lacking the PDZ domain) (55) mouse lines were acquired from Jackson Laboratories (Farmington, USA) and bred on a hybrid background: 50% 129SVPas - 50% C57BL/6J. *Fmr1*-KO2 mice (59) were generously provided by R. Willemsen (Erasmus University Medical Center, Rotterdam, The Netherlands) and bred on a C57BL/6J background. Equivalent numbers of male and female mice were generated in-house from homozygous parents, bred from heterozygous animals, to prevent genetic derivation. This breeding scheme favored social deficits in mutant mice by maintaining them together during early post-natal development. Except otherwise stated, animals were group-housed and maintained on a 12hr light/dark cycle (lights on at 7:00 AM) at controlled temperature (21±1°C); food and water were available *ad libitum.* Experiments were analyzed blind to genotypes and experimental condition. All experimental procedures were conducted in accordance with the European Communities Council Directive 2010/63/EU and approved by the Comité d'Ethique en Expérimentation animale Val de Loire (C2EA-19).

### Drugs

Mice were treated with vehicle (NaCl 0.9%; ip, 10 or 20 ml/kg), NaBr (Sigma-Aldrich, Saint-Quentin Fallavier, France) administered either chronically (once a day, 10, 30, 70, 125, 250 and 500 mg/kg; i.p. or per os, in a volume of 20 ml/kg - except for combination with VU0155041: 10 mg/kg) or acutely (250 mg/kg, 20 ml/kg), KBr (Sigma-Aldrich, Saint-Quentin Fallavier, France; 145 mg/kg; i.p., 20 ml/kg), bumetanide (R&D systems, Minneapolis, USA, 0.5 and 2 mg/kg; i.p., 20 ml/kg) or VU0155041 (Cayman Chemicals, Ann Arbor, USA, once a day, i.p., 1 mg/kg, 10 ml/kg). Doses of bumetanide were chosen based on previous studies in rodent models of ASD (*40, 73*); liminal dose of VU0155041 was set based on our previous studies (*51, 60*) and a pilot experiment showing no detectable effect in the social interaction test. When treatment was given chronically, behavioral testing started 8 days after beginning of daily administration. Treatment was maintained for 8 to 18 consecutive days (see timelines in **Figure 1** **and** **4**), allowing thorough behavioral phenotyping. On testing days, or when treatment was given acutely, drugs (or vehicle) were administered 30 min before behavioral assays.

### Behavioral experiments

When assessing effects of chronic administration, experiments were performed successively (timelines in **Figure 1** **and** **4****)** *(51,60).* Testing order was chosen to minimize the incidence of anxiety on later assays. Direct social interaction and novelty suppressed feeding were performed in 4 equal square arenas (open fields, 50 x 50 cm) separated by 35cm-high opaque grey Plexiglas walls over a white Plexiglas platform (View Point, Lyon, France). Stimulus mice used for the three-chamber test were 8-14-week-old grouped-housed male or female wild-type mice, socially naive to the experimental animals.

### Social abilities

**Direct social interaction test.** On testing day, a pair of unfamiliar mice (not cage mates, age-, sex- and treatment-matched) was introduced in each arena for 10 min (15 lx). Each arena received a black plastic floor (transparent to infrared). The total amount of time spent in nose contact (nose-to-nose, nose-to-body or nose-to-anogenital region), the number of these contacts, the time spent in paw contact and the number of these contacts, grooming episodes (allogrooming), notably ones occurring immediately (<5s) after a social contact, as well as the number of following episodes were scored *a posteriori* on video recordings (infrared light-sensitive video camera) using an ethological keyboard (Labwatcher^{®}, View Point, Lyon, France) by trained experimenters and individually for each animal (*51,60*)*.* The mean duration of nose and paw contacts was calculated from previous data (*61-63*).

**Three-chamber social preference test.** The test apparatus consisted of a transparent acrylic box (exterior walls blinded with black plastic film); partitions divided the box into three equal chambers (40 x 20 x 22.5 cm). Two sliding doors (8 x 5 cm) allowed transitions between chambers. Cylindrical wire cages (18 x 9 cm, 0.5 cm diameter-rods spaced 1 cm apart) were used to contain the mouse interactor and object (soft-toy mouse). The test was performed in low-light conditions (15 lx) to minor anxiety. Stimulus wild-type mice were habituated to confinement in wire cages for 2 days before the test (20 min/day). On testing day, the experimental animal was introduced to the middle chamber and allowed to explore the whole apparatus for a 10-min habituation phase (wire cages empty) after the sliding doors were raised. The experimental mouse was then confined back in the middle-chamber while the experimenter introduced an unfamiliar wild type age and sex-matched animal into a wire cage in one of the side-chambers and a soft toy mouse (8 x 10 cm) in the second wire cage as a control for novelty. Then the experimental mouse was allowed to explore the apparatus for a 10-min interaction phase. The time spent in each chamber, the time spent in nose contact with each wire cage (empty: habituation; containing a mouse or a toy: interaction), as well as the number of these nose contacts were scored *a posteriori* on video recordings using an ethological keyboard (Labwatcher^{®}, View Point, Lyon, France) by trained experimenters. The mean duration of nose contacts was calculated from these data (*61-63*). The relative position of stimulus mice (versus toy) was counterbalanced between groups.

### Stereotyped behaviors

**Motor stereotypies.** To detect motor stereotypies in mutant versus wild-type animals, mice were individually placed in clear standard home cages (21×11×17 cm) filled with 3-cm deep fresh sawdust for 10 min (*64*). Light intensity was set at 30 lux. Trained experimenters scored numbers of head shakes, as well as rearing, burying, grooming, circling episodes and total time spent burying by direct observation.

**Y-maze exploration.** Spontaneous alternation behavior was used to assess perseverative behavior (*65-67*). Each Y-maze consisted of three connected Plexiglas arms (15x15x17 cm) covered with distinct wall patterns (15 lx). Floors were covered with lightly sprayed fresh sawdust to limit anxiety. Each mouse was placed at the center of a maze and allowed to freely explore this environment for 5 min. The pattern of entries into each arm was quoted on video-recordings. Spontaneous alternations (SPA), i.e. successive entries into each arm forming overlapping triplet sets, alternate arm returns (AAR) and same arm returns (SAR) were scored, and the percentage of SPA, AAR and SAR was calculated as following: total / (total arm entries -2) * 100.

**Marble-burying.** Marble burying was used as a measure of perseverative behavior (68). Mice were introduced individually in transparent cages (21×11×17 cm) containing 20 glass marbles (diameter: 1.5 cm) evenly spaced on 4-cm deep fresh sawdust. To prevent escapes, each cage was covered with a filtering lid. Light intensity in the room was set at 40 lux. The animals were removed from the cages after 15 min, and the number of marbles buried more than half in sawdust was quoted.

### Anxiety-like behavior

**Novelty-suppressed feeding.** Novelty-suppressed feeding (NSF) was measured in 24-hr food-deprived mice, isolated in a standard housing cage for 30 min before individual testing. Three pellets of ordinary lab chow were placed on a white tissue in the center of each arena, lit at 60 lx. Each mouse was placed in a corner of an arena and allowed to explore for a maximum of 15 min. Latency to feed was measured as the time necessary to bite a food pellet. Immediately after an eating event, the mouse was transferred back to home cage (free from cage-mates) and allowed to feed on lab chow for 5 min. Food consumption in the home cage was measured.

### Nociceptive thresholds

**Tail-immersion test.** Nociceptive thresholds were assessed by immersing the tail of the mice (5 cm from the tip) successively into water baths at 48°C, 50°C and 52°C. The latency to withdraw the tail was measured at each temperature, with a cutoff of 10 s.

### Real-time quantitative PCR analysis

Brains were removed and placed into a brain matrix (ASI Instruments, Warren, MI, USA). Nucleus accumbens (NAc), caudate putamen (CPu), ventral pallidum/olfactory tubercle (VP/Tu), medial nucleus of the amygdala (MeA) and ventral tegmental area/substancia nigra pars compacta (VTA/SNc) were punched out/dissected from 1mm-thick slices **(data not shown).** Tissues were immediately frozen on dry ice and kept at -80°C until use. For each structure of interest, genotype and condition, samples were processed individually (n=8). RNA was extracted and purified using the Direct-Zol RNA MiniPrep kit (Zymo research, Irvine, USA). cDNA was synthetized using the ProtoScript II Reverse Transcriptase kit (New England BioLabs, Évry-Courcouronnes, France). qRT-PCR was performed in quadruplets on a CFX384 Touch Real-Time PCR Detection System (Biorad, Mames-la-Coquette, France) using iQ-SYBR Green supermix (Bio-Rad) kit with 0.25 µl cDNA in a 12 µl final volume in Hard-Shell Thin-Wall 384-Well Skirted PCR Plates (Bio-rad). Gene-specific primers were designed using Primer3 software to obtain a 100- to 150-bp product. Relative expression ratios were normalized to the level of actin and the 2^{-ΔΔCt} method was applied to evaluate differential expression level. Gene expression values differing of the mean by more than two standard deviations were considered as outliers and excluded from further calculations.

### Cell culture and transfection

Human embryonic kidney (HEK) 293 cells were transiently transfected with rat mGlu4 receptor by electroporation together with a chimeric Gi/Gq protein to allow phospholipase activation and EAAC1, a glutamate transporter, to avoid influence of extracellular glutamate. For calcium mobilization, cells were seeded in a PLO-coated, black-walled, clear-bottomed, 96-well plate (Greiner Bio-One) at the density of 100,000 cells per well and for IPOne assay in a PLO-coated black 96-well plate (Greiner Bio-One) at the density of 50,000 cells per well. Cells were cultured in DMEM (Gibco^{™}, Life Technologies), supplemented with 10% fetal bovine serum. Medium was changed by GlutaMAX (Gibco^{™}, Life Technologies) to reduce extracellular glutamate concentration 3 h before experiment.

### Chloride and bromide buffers for in vitro assays

Chloride and bromide ion concentrations in the buffers used for *in vitro* experiments were chosen for best matching physiological conditions. It was previously shown that the total amount of halogen (chloride and bromide ions) in the cerebrospinal fluid and serum can reach 120-130 mM (*69, 70*) and that bromide is able to substitute up to 30% of chloride concentration (*71*), leading to a theoretical concentration of 91 mM chloride and 36 mM bromide. We thus chose the dose of 100 mM chloride as physiological control (complemented with gluconate NaC₆H₁₁O₇ to maintain equivalent osmolarity between buffers (*56*)), and added or not 50 mM chloride or bromide to assess their effects *in vitro.* Chemicals were purchased from Sigma-Aldrich (Merck, L'lsle D'Abeau Chesnes, France). The pH of all buffers was adjusted to 7.4 before experiments.

For calcium mobilization assay, buffers with 100 mM NaCl, 2.6 mM KCl, 1.18 mM MgSO₄, 10 mM D-glucose, 10 mM 4-(2-hydroxyethyl)-1-piperazineethanesulfonic acid (HEPES), 1mM CaCl₂ and 0.5% (w/v) bovine serum albumin were used and supplemented with 50 mM of either NaCl, NaBr or NaC₆H₁₁O₇ to reach 154.6 mM total anion concentration.

For IP₁ accumulation assay, buffers with 46 mM NaCl, 4.2 mM KCl, 0.5 mM MgCl₂, 10 mM HEPES, 1mM CaCl₂, 50 mM NaC₆H₁₁O₇ and 50 mM LiCl (to avoid IP₁ degradation) were used and supplemented with 50 mM of either NaCl, NaBr or NaC₆H₁₁O₇ to reach 203.2 mM total anion concentration.

### Calcium mobilization and IP₁ accumulation assays

Calcium mobilization assay: 24 h after transfection, cells were loaded with 1 µM calcium-sensitive fluorescent dye (Fluo-4 AM; Invitrogen, Life Technologies) diluted in fresh Cl⁻ buffer (154.6 mM) for 1 h at 37°C and 5% CO₂. Then, cells were washed and maintained in appropriate buffer supplemented with 4 mM probenecid. Agonists were also diluted in appropriate buffers. Ca²⁺ release was determined using µCell FDSS (Hamamatsu Photonics). Fluoresence was recorded for 60s at 480 nm excitation and 540 nm emission after 20 s record of baseline.

IP₁ accumulation assay: Inositol monophosphate accumulation was determined using the IP-One HTRF (Homogenous Time Resolved Fluorescence) kit (Cisbio Bioassays, Perkin Elmer, Codolet, France) according to the manufacturer's recommendations (72). Briefly, cells were stimulated to induce IP₁ accumulation while being treated with test compounds in appropriate buffer for 30 min, at 37 °C and 5% CO2 before d2-labeled IP₁ and Tb-labeled anti-IP₁ antibody addition. After 1h incubation at RT, Pherastar FS (BMG Labtech) was used to record 620 nm and 665 nm emission after 337 nm excitation.

### Data analysis and statistics

### In vivo experiments

Statistical analyses were performed using Statistica 9.0 software (StatSoft, Maisons-Alfort, France). For all comparisons, values of p<0.05 were considered as significant. Statistical significance in behavioral experiments was assessed using one or two-way analysis of variance (drug, stimulus and treatment effects) followed by Newman-Keuls post-hoc test. Significance of quantitative real-time PCR (qRT-PCR) results was assessed after transformation using a two-tailed t-test, as previously described (*60*). Unsupervised clustering analysis was performed on transformed qRT-PCR data using complete linkage with correlation distance (Pearson correlation) for genotype and treatment (Cluster 3.0 and Treeview software) (*51, 60*). When used for clustering analysis **(data not shown),** behavioral data were normalized to vehicle-vehicle condition and transformed using the same formula as qRT-PCR data.

### In vitro experiments

Data were analyzed using Prism 6 software (GraphPad Software, San Diego, CA, USA). For IP₁ accumulation assay, each HTRF-ratio was transformed in IP₁ concentration using calibration curves for each buffer and then normalized against Mock-transfected cells to avoid buffer composition effect on HTRF signal. In all experiments, a 4-parameter concentration-response curve equation was used to fit data, and potency (EC₅₀) was estimated as logarithms (log EC₅₀). For clarity purpose, absolute (positive) logarithms (pEC₅₀) were used. Eₘₐₓ represents the maximum response obtained at saturating agonist concentration. Data shown in the figures represent the means ± SEM of at least 3 experiments realized in triplicates. Statistical differences between pEC₅₀, ΔpEC₅₀ and Eₘₐₓ were determined using a one-way analysis of variance followed by Tukey's post-test.

### Results

### Chronic sodium bromide was more efficient than bumetanide to relieve social behavior deficits in Oprm1^{-/-} mice

We first assessed the effects of NaBr administration over a wide range of doses (10 to 500 mg/kg) in *Oprm1*^{*-*/*-*} mice and their WT counterparts, and compared with bumetanide administration (0.5 and 2 mg/kg) **(****Figure 1A****).** Treatment was given chronically to mimic clinical conditions.

Social interaction was evaluated after 9 days of chronic NaBr treatment **(Figure 1B1-1B2).** *Oprm1*^{*-*/*-*} mice exhibited a severe decrease in social interaction; chronic NaBr administration from the dose of 125 mg/kg dose-dependently relieved this deficit in mutant mice, as evidenced by restored number *(genotype x treatment: F_{6,163}=13.2, p<0.0001)* and mean duration *(genotype x treatment: F_{6,163}=31.6, p<0.0001)* of nose contacts and normalized number *(genotype x treatment: F_{6,163}=14.7, p<0.0001)* and mean duration *(genotype x treatment: F_{6,163}=14.5, p<0.0001)* of paw contacts. Chronic NaBr also increased the number of following episodes in both mouse lines *(treatment: F_{6,163}=5.6, p<0.0001)* and normalized the frequency of grooming after social contact in mutants, since the dose of 70 mg/kg *(genotype x treatment: F_{6,163}*=32.2*,p<0.0001*)*.* In contrast, a single acute injection ofNaBr (250 mg/kg) had little effect on social interaction parameters **(data not shown).** When given chronically, NaBr (over 70 mg/kg) produced relieving effects that were still detectable one week after cessation of treatment, as evidenced by preserved restoration of the duration of nose contacts *(genotype x treatment: F_{6,134}*=53.3, *p<0.0001)* and maintained suppression of grooming after social contact *(genotype x treatment: F_{6,134}*=30.4, *p<0.0001*) for the highest doses **(****Figure 1C****).** These effects had mostly vanished after two weeks **(data not shown).**

Compared with chronic NaBr, chronic bumetanide increased the number of nose contacts *(genotype x treatment: F_{2,42}=22.6, p<0.0001)* and following episodes at low dose *(genotype x treatment: F_{2,42}=12.9, p<0.0001)* but failed to increase significantly the duration of nose contacts *(genotype x treatment: F_{2,42}=22.6, p<0.0001)* or the number *(genotype x treatment: F_{2,42}=14.9, p<0.0001)* and duration *(genotype x treatment: F_{2,42}=7.3, p<0.0001)* of paw contacts. Finally, bumetanide suppressed grooming episodes, notably those occurring after social contact, since the lowest dose tested *(genotype x treatment: F_{2,42}=80.7, p<0.0001).* Of note, chronic bumetanide treatment showed deleterious effects on social interaction parameters in WT controls. One week after cessation of treatment, beneficial effects of bumetanide were still detectable notably on the duration of nose contacts *(genotype x treatment: F_{2,42}=9.3, p<0.0001)* and grooming episodes after social contact *(genotype x treatment: F_{2,42}=16.4, p<0.0001),* depending on the dose **(****Figure 1C****).**

In the 3-chamber test **(****Figure 1D****),** *Oprm1*^{*-*/*-*} mice showed a severe impairment in social preference, as evidenced by equivalent number of nose contacts made with the mouse and the toy, and even longer nose contacts made with the toy over the mouse. Chronic NaBr completely restored social preference in mutant mice, which displayed more frequent (*genotype x treatment x stimulus: F_{6,160}= 3.5, p<0.001)* and longer *(genotype x treatment x stimulus: F_{6,160}= 8.9, p<0.0001)* nose contacts with the mouse since the lowest dose of bromide administered. This resulted in a normalization of their preference ratio from 10 mg/kg NaBr and over *(genotype x treatment: F_{6,160}=11.9, p<0.0001*). In this test, chronic bumetanide at the lowest dose restored a preference for making more frequent nose contacts with the mouse over the toy *(genotype x treatment x stimulus: F_{2,42}=17.7, p<0.0001).* Bumetanide treatment in mutant mice dose-dependently increased the duration of nose contacts with the mouse, but failed to reduce the duration of nose contacts with the toy *(genotype x treatment x stimulus: F_{2,42}=32.5, p<0.0001)* leading to significant but partial recovery of social preference ratio in *Oprm1*^{*-*/*-*} mice (*genotype x treatment: F_{2,42}= 38.4, p<0.0001).*

In conclusion, chronic but not acute NaBr treatment restored social behavior in *Oprm1*^{*-*/*-*} mice in a dose-dependent manner, and these beneficial effects were superior to those of chronic bumetanide treatment.

### Sodium bromide reduced stereotypic behaviors and anxiety in Oprm1^{-/-} mice

We next assessed the effects of chronic bromide on non-social behaviors in the same cohorts of *Oprm1*^{*-*/*-*} mice (timeline in **Figure 1A****).**

Regarding stereotypic behavior, *Oprm1*^{*-*/*-*} mice displayed spontaneous stereotypic circling and head shakes **(Figure 2A1-A2)** that were decreased under NaBr treatment since the lowest dose, more consistently for the former *(genotype x treatment: F_{6,161}=4.6, p<0.001)* than for the latter *(genotype x treatment: F_{6,161}=7.0, p<0.0001).* In *Oprm1*^{*+*/*+*} control mice, NaBr dose-dependently increased the number of grooming episodes *(genotype x treatment: F_{6,161}=4.5, p<0.001)* and head shakes *(genotype x treatment: F_{6,161}=7.0, p<0.0001);* in both mouse lines, NaBr increased the number of rearing episodes in a dose-dependent manner *(treatment: F_{6,161}=13.2, p<0.0001).* Under the same conditions, bumetanide suppressed circling and head shakes in mutant mice, and reduced the number of rearing episodes in both mouse lines. In the marble burying test **(****Figure 2B****),** NaBr treatment did not suppressed excessive burying in mutant mice *(genotype: F_{6,163}=13.6, p<0.001)* and globally increased burying *(treatment: F_{6,163}=2.4, p<0.05).* Similarly, chronic bumetanide failed to suppress excessive marble burying in *Oprm1*^{*-*/*-*} mice *(genotype: F_{2,46}=14.7, p<0.001).* In the Y-maze exploration test **(****Figure 2C****),** bromide decreased the number of perseverative same arm returns in *Oprm1* null mice to wild-type levels since the dose of 30 mg/kg *(genotype x treatment: F_{6,161}=6.7, p<0.0001)* while bumetanide globally failed to suppress those *(genotype: F_{2,46}=19.7, p<0.001),* despite a tendency for a decrease observed for the dose of 0.5 mg/kg.

We assessed anxiety levels in *Oprm1*^{*-*/*-*} mice and their WT counterparts using the novelty suppressed feeding test **(****Figure 2D****).** Mutant mice displayed exaggerated anxiety in this test, with increased latency to eat and reduced food intake once back in their home cage. Chronic bromide normalized eating latency *(genotype x treatment: F_{6,160}=6.7, p<0.0001)* to wild-type levels in *Oprm1* knockout mice and increased food intake in both mouse lines *(treatment: F_{6,160}=7.0, p<0.0001)* since the lowest dose tested. Chronic bumetanide had no detectable effect on either latency to eat *(genotype: F_{2,42}=27.1, p<0.0001)* or food intake *(genotype: F_{2,42}=5.6, p<0.05)* in this test.

Together, these results indicate that chronic bromide and bumetanide treatments both reduced stereotypic behavior in *Oprm1*^{*-*/*-*} mice but only bromide treatment demonstrated anxiolytic effects.

In a next series of experiments, we verified that NaBr administered via oral route (oral gavage 4-5 days, 250 mg/kg once per day) relieved autistic-like deficits and motor stereotypies in *Oprm1*^{*-*/*-*} mice in a similar way as it did after intra-peritoneal injection **(data not shown).** Also, we assessed the behavioral effects of chronic administration of another bromide salt, KBr. A dose of KBr equivalent to 250 mg/kg NaBr being toxic in pilot experiments, we thus lowered the dose to 145 mg/kg KBr, equivalent to 125 mg/kg NaBr. Beneficial effects of NaBr treatment in *Oprm1*^{*-*/*-*} mice were fully replicated, if not exceeded, by KBr in tests assessing social, repetitive and anxious behavior **(data not shown).** Thus, therapeutic effects of NaBr or KBr in *Oprm1*^{*-*/*-*} mice were attributable to bromide ions.

### Chronic sodium bromide relieved social behavior deficits, stereotypies and excessive anxiety in Fmr1^{-/-} and Shank3^{Δex13-16-/-} mice

We then questioned whether beneficial effects of NaBr on autistic-like symptoms may generalize to other mouse models of ASD, here the *Fmr1* null and *Shank3*^{Δ*ex13-16*} knockout mouse lines. To this purpose, we evaluated the effects of chronic NaBr administration at the dose of 250 mg/kg on autism-sensitive behaviors in these lines **(****Figure 3A****).**

As concerns social behavior, during a direct social interaction test **(****Figure 3B****),** chronic bromide in *Fmr1*^{*-*/*-*} as well as *Shank3*^{Δ*ex13-16-*/*-*} mice restored the duration of nose *(genotype x treatment - Fmr1: F_{1,30}=49.5, p<0.0001; Shank3*^{Δ}*^{ex13-16}: F_{1,28}=73.0, p<0.0001)* and paw contacts *(genotype x treatment- Fmr1: F_{1.30}=17.7, p<0.0001; Shank3*^{Δ}*^{ex13-16}: F_{1,28}=23.2, p<0.0001),* normalized the number of following episodes *(genotype x treatment - Fmr1: F_{1,30}=13.7, p<0.0001; Shank3*^{Δ}*^{ex13-16}: F_{1,28}=11.8, p<0.0001)* and suppressed grooming after social contact *(genotype x treatment- Fmr1: F_{1,30}=30.1, p<0.0001; Shank3*^{Δ}*^{ex13-16}: F_{1,28}=25.0, p<0.0001).* One week after interruption of NaBr treatment **(****Figure 3C****),** significant beneficial effects were still detected on the duration of paw contacts *(genotype x treatment: F_{1,30}=132.7, p<0.0001)* and number of grooming episodes after a social contact *(genotype x treatment: F_{1,30}=87.0, p<0. 0001)* in *Fmr1* null mice. In *Shank3*^{Δ*ex13-16*} mice, no effect of previous bromide treatment was longer detected on the duration of paw contacts *(genotype: F_{1,28}=388.2, p<0.0001),* whereas grooming after social contact remained efficiently suppressed *(genotype x treatment: F_{1,28}=55.3, p<0.0001).*

We further assessed social behavior under chronic bromide exposure using the 3-chamber test **(****Figure 3D****).** Although *Fmr1* knockout mice made more frequent nose contacts with the mouse versus the object in this test, they spent as much time in contact with the living mouse as with the object and made longer nose contacts with the object, which demonstrates disrupted social preference. Chronic NaBr treatment restored a preference for spending more time exploring the mouse *(genotype x treatment x stimulus: F_{1,29}= 4.5, p<0.05)* and making longer nose contacts with the mouse versus the object *(genotype x treatment x stimulus: F_{1,29}= 18.4, p<0.001)* in *Fmr1* mutants, which normalized their preference ratio *(genotype x treatment: F_{1,29}=5.2, p<0.05)* without modifying the number of nose contacts they made with either stimulus *(stimulus: F_{1,29}=21.4, p<0.0001).* Likewise, *Shank3*^{Δ*ex13-16-*/*-*} mice treated with vehicle failed to spend more time with the mouse over the toy in this test; they made more frequent nose contacts with the mouse but of equivalent duration with both stimuli. In contrast, mutants treated with NaBr spent more time in contact with the mouse (*genotype x treatment x stimulus: F_{1,28}= 4.5, p<0.05*) and made longer nose contacts with their congener versus the toy (*genotype x treatment x stimulus: F_{1,28}= 26.9, p<0.0001)*, resulting in increased preference ratio (*genotype x treatment: F_{1,28}=5.9, p<0.05)* with no change in the number of nose contacts (*stimulus: F_{1,28}=37.4, p<0.0001).* Thus, chronic NaBr administration rescued social behavior deficits in *Fmr1* null and *Shank3*^{Δ*ex13-16*} knockout mice.

As regards stereotypic behavior, *Fmr1*^{*-*/*-*} and *Shank3*^{Δ}*^{ex13-1}6*^{*-*/*-*} mice displayed more frequent spontaneous grooming (significant in the latter only), circling episodes and head shakes than WT controls **(****Figure 3E****).** Chronic NaBr treatment normalized all these parameters to WT levels (*Fmr1 - circling, genotype x treatment: F_{1,30}=11.9, p<0.01; head shakes, genotype x treatment: F_{1,30}=68.0, p<0.001; Shank3*^{Δ}*^{ex13-16} - grooming, genotype x treatment: F_{1,28}=10.2, p<0.01, circling, genotype x treatment: F_{1,28}=48.4, p<0.0001; head shakes, genotype x treatment: F_{1,28}=5.3, p<0.05).* In the marble burying test **(****Figure 3F****),** chronic NaBr reduced the number of buried marbles in both *Fmr1* ^{*+*/*+*} and *Fmr1*^{*-*/*-*} mice *(treatment effect: F_{1,30}=7.3, p<0.05); Shank3*^{Δ*ex13-16-*/*-*} mice displayed a severe deficit in marble burying (*genotype effect: F_{1,28}=198.7, p<0.0001)* and bromide treatment decreased burying in both mutant and WT mice *(treatment effect: F_{1,28}=4.8, p<0.05).* In the Y-maze **(****Figure 3G****),** *Fmr1* null and *Shank3*^{Δ*ex13-16*} knockout mice displayed more frequent perseverative same arm returns that were suppressed under chronic bromide (*Fmr1 - genotype x treatment: F_{1,30}=61.9, p<0.0001; Shank3*^{Δ}*^{ex13-16} - genotype x treatment: F_{1,28}=47.5, p<0.0001).* Thus, chronic NaBr treatment reduced stereotypic and perseverative behaviors in *Fmr1*^{*-*/*-*} and*Shank3*^{Δ*ex13-16-*/*-*} mice.

As regards anxiety, a tendency for *Fmr1* null mice for increased latency to eat in the novelty-suppressed feeding test **(****Figure 3H****)** did not reach significance; however chronic bromide reduced this latency *(genotype x treatment: F_{1,30}=6.7, p<0.05). Shank3*^{Δ*ex13-16-*/*-*} mice took significantly longer to eat in the center of the arena and NaBr administration normalized this latency to WT levels *(genotype x treatment: F_{1,28}=35.0, p<0.0001).* Bromide treatment had no effect on food intake, reduced in *Shank3*^{Δ*ex13-16*} knockout mice *(genotype: F_{1,28}=15.6, p<0.001).* Therefore, chronic NaBr administration demonstrated anxiolytic properties in *Fmr1*^{*-*/*-*} and *Shank3*^{Δ*ex13-16-*/*-*} mice.

### Chronic sodium bromide modulates transcription in the reward circuit of Oprm1^{-/-} mice

To shed light on the molecular mechanism involved in beneficial effects of chronic sodium bromide administration, we assessed the effects of a 2-week NaBr treatment on gene expression in *Oprm1* null mice across five regions of the brain reward/social circuit: NAc, CPu, VP/Tu, MeA and VTA/SNc. Mice underwent a first session of social interaction after one week under treatment and a second 45 min before sacrifice for qRT-PCR experiment **(data not shown).** We focused primarily on genes coding for chloride transporters (*Slc12a2* [NKCC1], *Slc12a4,5,6,7* [KCC1,2,3,4, respectively], *ClCa1*), GABA_{A} receptor subunits (*Gabra1,2,3,4,5, Gabrb1,2*) and glutamate receptors (*Grm2,4,5*) and subunits (*Grin2*a*,2b*). In addition, we evaluated the expression of marker genes of neuronal expression and plasticity (*Fos, Bdnf*), social behavior (*Oxt*) and striatal projection neurons (SPNs; *Crh, Drd1a, Drd2, Htr6, Pdyn, Penk).*

We performed hierarchical clustering analysis of qRT-PCR data for each brain region to visualize the influence of NaBr treatment on gene expression **(data not shown).** Overall, transcriptional profiles in *Oprm1*^{*-*/*-*} mice under vehicle and NaBr treatment differed the most, while mRNA levels correlated poorly with social interaction parameters **(data not shown).** These results indicate that bromide induced transcriptional changes on its own rather than it normalized gene expression in *Oprm1* knockouts (as observed for behavioral parameters). This was particularly true in the CPu, where *Oprm1*^{*-*/*-*} mice under bromide treatment displayed predominant up-regulated gene expression (clusters a and c).

This overall profile was confirmed when focusing on candidate genes **(data not shown).** We ought to acknowledge here that the sample number of mice allocated to each experimental condition was low to address the complex influences of genotype and pharmacological treatment, which may have limited the statistical power. For this reason, we focused our attention on gene expression regulations affecting either several brain regions for the same gene, or several genes of the same family. Strikingly, chronic NaBr administration increased the expression of all tested chloride transporters in *Oprm1*^{*-*/*-*} mice. Indeed, the expression of *Slc12a2* was decreased in all brain regions but the VP/Tu of mutant mice, and normalized under bromide treatment. Chronic NaBr up-regulated the expression of *Slc12a5* and *Slc12a7* in the NAc, CPu and MeA of *Oprm1* null mice for the former, together with the VP/Tu for the latter. In mutant mice, *ClCa1* mRNA levels were increased in the NAc, MeA and VTA/SNc while reduced in the VP/Tu; they were normalized by NaBr treatment in the NAc, increased in the VP/Tu and maintained high in the MeA and VTA/SNc. In the CPu, bromide increased *ClCa1* transcription in mice of both genotypes. As regards the GABAergic system, chronic NaBr in *Oprm1*^{*-*/*-*} mice stimulated the expression of *Gabra2,* coding for the α2 subunit of the GABA_{A} receptor, in the NAc and CPu and left this expression high in the VP/TU and MeA. Remarkably, bromide consistently upregulated the expression of *Gabra3, Gabra4, Gabra5, Gabrg1* and *Gabrb2* in the CPu of mutant mice **(data not shown).** Bromide treatment down-regulated the expression of the early gene *Fos* in the NAc and VP/Tu and maintained it low in the CPu of *Oprm1* knockouts. *Oxt* (coding for oxytocin) mRNA levels were decreased in the NAc and VP/Tu of *Oprm1*^{*-*/*-*} mice and normalized by NaBr in the former and partially in the latter; bromide induced *Oxt* expression in the MeA and VTA/SNc. Finally, chronic NaBr upregulated the expression of *Grm4,* coding for the metabolic glutamate receptor mGlu4, in all brain regions but the VTA/SNc of mutant mice, and in the NAc of wild-type controls. Transcriptional results thus indicate that bromide administration had a major impact on the expression of Cl⁻ transporters; meanwhile it also regulated the expression of several key players of the GABA system, marker genes of neuronal activity and plasticity, and genes more specifically involved in the control of social behavior.

### Synergistic effects of chronic bromide and mGlu4 receptor facilitation in Oprm1 null mice

Intrigued by the increase in *Grm4* transcription in *Oprm1*^{*-*/*-*} mice under bromide treatment, whose autistic-like symptoms were relieved when stimulating mGlu4 activity (*51*), we then addressed the question of a potential shared mechanism of action between these treatments. To this aim, we studied the effects of combined administration of chronic liminal doses of NaBr (70 mg/kg) and VU0155041 (1 mg/kg) in *Oprm1*^{*-*/*-*} and *Oprm1*^{*+*/*+*} mice **(****Figure 4A****).**

In the direct social interaction test **(****Figure 4B****),** NaBr at 70 mg/kg had no detectable effects on behavioral parameters in *Oprm1*^{*-*/*-*} mice (see **Figure 1B);** VU0155041 partially rescued the duration of their nose contacts and suppressed grooming episodes after a social contact. When the two treatments were combined, social interactions parameters in mutant mice were normalized to wild-type levels, as illustrated by rescued duration of nose contacts *(genotype x bromide x VU0155041: F_{1,56}=31.1, p<0.0001)* or paw contacts (*genotype x bromide x VU0155041: F_{1,56}=140.3, p<0.0001*) and normalized number of grooming episodes after social contact *(genotype x bromide x VU0155041: F_{1,56}=5.8, p<0.05).* Restoration of the mean duration of nose contacts was fully preserved one (*genotype x bromide x VU0155041: F_{1,56}=40.9, p<0.0001)* and two *(genotype x bromide x VU0155041: F_{1,56}=57.3, p<0.0001)* weeks after cessation of treatment. In the 3-chamber test, NaBr at 70 mg/kg or VU0155041 at 1 mg/kg were sufficient to restore more frequent nose contacts with the mouse versus the toy in *Oprm1*^{*-*/*-*} mice *(genotype x NaBr x VU0155041x stimulus: F_{1,55}=6.7, p<0.05)* **(****Figure 4C****).**

As regards stereotypic behavior, combined NaBr and VU0155041 treatments reduced the number of head shakes in *Oprm1*^{*-*/*-*} and *Oprm1*^{*+*/*+*} mice *(bromide x VU0155041: F_{1,56}=4.1, p<0.05*) **(****Figure 4D****)** and normalized marble burying in mutants *(genotype x NaBr x VU0155041: F_{1,56}=9.5, p<0.001)* **(****Figure 4E****).** Chronic VU0155041 was sufficient to suppress perseverative same arm returns during Y-maze exploration in *Oprm1* null mice *(genotype x VU0155041: F_{1,56}=11.3, p<0.01)* **(****Figure 4F****).** In the novelty-suppressed feeding test, chronic NaBr was similarly sufficient to normalize latency to feed *(genotype x VU0155041: F_{1,56}=11.3, p<0.01)* **(****Figure 4G****).** Finally, having in mind that nociceptive thresholds are lowered in *Oprm1* null mice, we tested the effects of bromide and VU0155041 on this parameter. At 50°C, combined NaBr and VU0155041 treatment normalized flicking latency, while each compound given alone was ineffective *(genotype x NaBr x VU0155041: F_{1,56}=20.5, p<0.0001)* **(****Figure 4H****).**

Together, these results indicate that bromide administration and facilitation of mGlu4 activity exert synergistic beneficial effects on autistic-like behavior in *Oprm1*^{*-*/*-*} mice.

### Bromide ions behave as positive allosteric modulators of the mGlu4 glutamate receptor

Chloride ions have been shown to facilitate mGlu4 signaling (56). Here we assessed whether synergistic *in vivo* effects of bromide treatment and VU0155041 administration may result from a modulation of mGlu4 activity by bromide ions, in addition to their ability to trigger an upregulation of *Grm4* expression **(data not shown).**

We measured mGlu4 signaling under glutamate stimulation in HEK293T cells transiently expressing mGlu4 receptors and Gα_{qi9}, a Gᵢ/G_{q} chimeric G-protein (57) that allows mGlu4 to activate the phosphoinositide pathway. Receptor activation was then evaluated by measuring intracellular Ca²⁺ release or inositol monophosphate (IP₁, **Figure 5A**). The experiments were performed in buffers containing either a physiological concentration of chloride ions (100 mM, supplemented with 50 mM gluconate to maintain equivalent osmolarity between mediums), 150 mM of chloride ions (classical buffer for cell culture studies) or 100 mM of chloride ions and 50 mM of bromide ions, to compare the effects of modulating chloride and bromide concentrations within a physiological range on mGlu4 activity.

Compared with physiological concentration of chloride (100 mM Cl⁻ + 50 mM gluconate), addition of 50 nM bromide significantly improved glutamate potency, showing a 0.73 ± 0.05 log increase in pEC₅₀ (left panel; *ion concentration: F_{2,6}=104.2, p<0.0001)* in the Ca²⁺ assay **(****Figure 5B****).** Compared with an equivalent concentration of chloride (+ 50 mM Cl⁻), bromide showed a higher efficacy (ΔpEC₅₀: 0.27±0.04) (right panel; *ion concentration: F_{2,6}=66.7, p<0.0001).* Further, bromide increased glutamate efficacy, with a 65 ± 9% rise in maximal mGlu4-triggered calcium release (Eₘₐₓ, left panel; *ion concentration: F_{2,21}=56.2, p<0.0001).*

When measuring IP₁ production **(****Figure 5C****),** bromide increased glutamate efficacy (Eₘₐₓ, maximal IP1 production) within a similar range (60 ± 10%) as observed when measuring Ca²⁺ release (left panel; *ion concentration: F_{2,24}=22.1, p<0.0001).* Despite technical limitations (less amplification at this step of the signaling cascade), pEC₅₀ was consistently increased in presence of bromide ions (left panel; *ion concentration: F_{2,6}=104.2, p<0.0001;* ΔpEC₅₀ between physiological Cl⁻ concentration and after addition of 50 nM Br⁻: 0.42 ± 0.03) though to a lower extend as those measured in the Ca²⁺ assay. When 5µM of VU0155041 were added, bromide significantly increased glutamate potency compared with physiological concentration of chloride (ΔpEC₅₀: 0.72 ± 0.03). Bromide and VU0155041 combination was also more effective in increasing glutamate potency than VU0155041 (ΔpEC₅₀: 0.39 ± 0.03) or bromide (ΔpEC₅₀: 0.30 ± 0.03) alone *(ion concentration and VU0155041: F_{5,12}=49.4, p<0.0001).*

In conclusion, bromide ions behaved as PAMs of the mGlu4 receptor in heterologous cells. These PAM effects were superior to those of chloride ions, and synergized with those of VU0155041. Together, these results provide a molecular mechanism for synergistic effects of bromide and VU0155041 in *Oprm1* null mice, and suggest that benefits of bromide treatment in mouse models of ASD involved, at least in part, a facilitation of mGlu4 activity.

### Conclusion

In conclusion, the present study reports the therapeutic potential of chronic bromide treatment, alone or in combination with a PAM of mGlu4 receptor, to relieve core symptoms of ASD. Beneficial effects of bromide were observed in three mouse models of ASD with different genetic causes, supporting high translational value. Moreover, bromide has a long history of medical use, meaning that its pharmacodynamics and toxicity are well known, which, combined with long lasting effects as well as excellent oral bioavailability and brain penetrance, are strong advantages for repurposing.

### REFERENCES:

Throughout this application, various references describe the state of the art to which this invention pertains.
1. APA, Diagnostic and statistical manual of mental disorders. (Washington, DC, ed. 5th ed., 2013).
2. C. P. Johnson, S. M. Myers, Identification and evaluation of children with autism spectrum disorders. Pediatrics 120, 1183-1215 (2007).
3. M. C. Lai, M. V. Lombardo, S. Baron-Cohen, Autism. Lancet 383, 896-910 (2014).
4. M. O. Mazurek, R. A. Vasa, L. G. Kalb, S. M. Kanne, D. Rosenberg, A. Keefer, D. S. Murray, B. Freedman, L. A. Lowery, Anxiety, sensory over-responsivity, and gastrointestinal problems in children with autism spectrum disorders. J Abnorm Child Psychol 41, 165-176 (2013).
5. E. Fombonne, L. Green Snyder, A. Daniels, P. Feliciano, W. Chung, S. Consortium, Psychiatric and Medical Profiles of Autistic Adults in the SPARK Cohort. J Autism Dev Disord 50, 3679-3698 (2020).
6. F. K. Satterstrom, J. A. Kosmicki, J. Wang, M. S. Breen, S. De Rubeis, J. Y. An, M. Peng, R. Collins, J. Grove, L. Klei, C. Stevens, J. Reichert, M. S. Mulhern, M. Artomov, S. Gerges, B. Sheppard, X. Xu, A. Bhaduri, U. Norman, H. Brand, G. Schwartz, R. Nguyen, E. E. Guerrero, C. Dias, C. Autism Sequencing, P.-B. C. i, C. Betancur, E. H. Cook, L. Gallagher, M. Gill, J. S. Sutcliffe, A. Thurm, M. E. Zwick, A. D. Borglum, M. W. State, A. E. Cicek, M. E. Talkowski, D. J. Cutler, B. Devlin, S. J. Sanders, K. Roeder, M. J. Daly, J. D. Buxbaum, Large-Scale Exome Sequencing Study Implicates Both Developmental and Functional Changes in the Neurobiology of Autism. Cell 180, 568-584 e523 (2020).
7. S. J. Sanders, M. T. Murtha, A. R. Gupta, J. D. Murdoch, M. J. Raubeson, A. J. Willsey, A. G. Ercan-Sencicek, N. M. DiLullo, N. N. Parikshak, J. L. Stein, M. F. Walker, G. T. Ober, N. A. Teran, Y. Song, P. El-Fishawy, R. C. Murtha, M. Choi, J. D. Overton, R. D. Bjornson, N. J. Carriero, K. A. Meyer, K. Bilguvar, S. M. Mane, N. Sestan, R. P. Lifton, M. Gunel, K. Roeder, D. H. Geschwind, B. Devlin, M. W. State, De novo mutations revealed by whole-exome sequencing are strongly associated with autism. Nature 485, 237-241 (2012).
8. H. R. Park, J. M. Lee, H. E. Moon, D. S. Lee, B. N. Kim, J. Kim, D. G. Kim, S. H. Paek, A Short Review on the Current Understanding of Autism Spectrum Disorders. Experimental neurobiology 25, 1-13 (2016).
9. E. Lee, J. Lee, E. Kim, Excitation/Inhibition Imbalance in Animal Models of Autism Spectrum Disorders. Biol Psychiatry 81, 838-847 (2017).
10. S. B. Nelson, V. Valakh, Excitatory/Inhibitory Balance and Circuit Homeostasis in Autism Spectrum Disorders. Neuron 87, 684-698 (2015).
11. J. L. Rubenstein, M. M. Merzenich, Model of autism: increased ratio of excitation/inhibition in key neural systems. Genes Brain Behav 2, 255-267 (2003).
12. G. Cellot, E. Cherubini, GABAergic signaling as therapeutic target for autism spectrum disorders. Front Pediatr 2, 70 (2014).
13. C. E. Robertson, E. M. Ratai, N. Kanwisher, Reduced GABAergic Action in the Autistic Brain. Current biology : CB 26, 80-85 (2016).
14. R. Muhle, S. V. Trentacoste, I. Rapin, The genetics of autism. Pediatrics 113, e472-486 (2004).
15. L. Cornew, T. P. Roberts, L. Blaskey, J. C. Edgar, Resting-state oscillatory activity in autism spectrum disorders. J Autism Dev Disord 42, 1884-1894 (2012).
16. J. A. Munoz-Yunta, T. Ortiz, M. Palau-Baduell, L. Martin-Munoz, B. Salvado-Salvado, A. Valls-Santasusana, J. Perich-Alsina, I. Cristobal, A. Fernandez, F. Maestu, C. Dursteler, Magnetoencephalographic pattern of epileptiform activity in children with early-onset autism spectrum disorders. Clinical neurophysiology : official journal of the International Federation of Clinical Neurophysiology 119, 626-634 (2008).
17. S. S. Jeste, R. Tuchman, Autism Spectrum Disorder and Epilepsy: Two Sides of the Same Coin? J Child Neurol 30, 1963-1971 (2015).
18. L. Strasser, M. Downes, J. Kung, J. H. Cross, M. De Haan, Prevalence and risk factors for autism spectrum disorder in epilepsy: a systematic review and meta-analysis. Dev Med Child Neurol 60, 19-29 (2018).
19. C. O'Donnell, J. T. Goncalves, C. Portera-Cailliau, T. J. Sejnowski, Beyond excitation/inhibition imbalance in multidimensional models of neural circuit changes in brain disorders. eLife 6, (2017).
20. T. Rinaldi, K. Kulangara, K. Antoniello, H. Markram, Elevated NMDA receptor levels and enhanced postsynaptic long-term potentiation induced by prenatal exposure to valproic acid. Proc Natl Acad Sci U S A 104, 13501-13506 (2007).
21. R. Shi, P. Redman, D. Ghose, H. Hwang, Y. Liu, X. Ren, L. J. Ding, M. Liu, K. J. Jones, W. Xu, Shank Proteins Differentially Regulate Synaptic Transmission. eNeuro 4, (2017).
22. L. K. Fung, R. E. Flores, M. Gu, K. L. Sun, D. James, R. K. Schuck, B. Jo, J. H. Park, B. C. Lee, J. H. Jung, S. E. Kim, M. Saggar, M. D. Sacchet, G. Wamock, M. M. Khalighi, D. Spielman, F. T. Chin, A. Y. Hardan, Thalamic and prefrontal GABA concentrations but not GABAA receptor densities are altered in high-functioning adults with autism spectrum disorder. Mol Psychiatry 26, 1634-1646 (2020).
23. S. H. Fatemi, T. J. Reutiman, T. D. Folsom, P. D. Thuras, GABA(A) receptor downregulation in brains of subjects with autism. J Autism Dev Disord 39, 223-230 (2009).
24. A. L. Oblak, T. T. Gibbs, G. J. Blatt, Decreased GABA(B) receptors in the cingulate cortex and fusiform gyrus in autism. J Neurochem 114, 1414-1423 (2010).
25. C. V. Sesarini, L. Costa, N. Granana, M. G. Coto, R. C. Pallia, P. F. Argibay, Association between GABA(A) receptor subunit polymorphisms and autism spectrum disorder (ASD). Psychiatry Res 229, 580-582 (2015).
26. M. Mahdavi, M. Kheirollahi, R. Riahi, F. Khorvash, M. Khorrami, M. Mirsafaie, Meta-Analysis of the Association between GABA Receptor Polymorphisms and Autism Spectrum Disorder (ASD). Journal of molecular neuroscience : MN 65, 1-9 (2018).
27. D. C. Adusei, L. K. Pacey, D. Chen, D. R. Hampson, Early developmental alterations in GABAergic protein expression in fragile X knockout mice. Neuropharmacology 59, 167-171 (2010).
28. A. Banerjee, F. Garcia-Oscos, S. Roychowdhury, L. C. Galindo, S. Hall, M. P. Kilgard, M. Atzori, Impairment of cortical GABAergic synaptic transmission in an environmental rat model of autism. The international journal of neuropsychopharmacology 16, 1309-1318 (2013).
29. H. T. Chao, H. Chen, R. C. Samaco, M. Xue, M. Chahrour, J. Yoo, J. L. Neul, S. Gong, H. C. Lu, N. Heintz, M. Ekker, J. L. Rubenstein, J. L. Noebels, C. Rosenmund, H. Y. Zoghbi, Dysfunction in GABA signalling mediates autism-like stereotypies and Rett syndrome phenotypes. Nature 468, 263-269 (2010).
30. G. Curia, T. Papouin, P. Seguela, M. Avoli, Downregulation of tonic GABAergic inhibition in a mouse model of fragile X syndrome. Cereb Cortex 19, 1515-1520 (2009).
31. S. Han, C. Tai, C. J. Jones, T. Scheuer, W. A. Catterall, Enhancement of inhibitory neurotransmission by GABAA receptors having alpha2,3-subunits ameliorates behavioral deficits in a mouse model of autism. Neuron 81, 1282-1289 (2014).
32. S. Han, C. Tai, R. E. Westenbroek, F. H. Yu, C. S. Cheah, G. B. Potter, J. L. Rubenstein, T. Scheuer, H. O. de la Iglesia, W. A. Catterall, Autistic-like behaviour in Scn1a+/- mice and rescue by enhanced GABA-mediated neurotransmission. Nature 489, 385-390 (2012).
33. C. Henderson, L. Wijetunge, M. N. Kinoshita, M. Shumway, R. S. Hammond, F. R. Postma, C. Brynczka, R. Rush, A. Thomas, R. Paylor, S. T. Warren, P. W. Vanderklish, P. C. Kind, R. L. Carpenter, M. F. Bear, A. M. Healy, Reversal of disease-related pathologies in the fragile X mouse model by selective activation of GABAB receptors with arbaclofen. Sci Transl Med 4, 152ra128 (2012).
34. J. L. Silverman, M. C. Pride, J. E. Hayes, K. R. Puhger, H. M. Butler-Struben, S. Baker, J. N. Crawley, GABAB Receptor Agonist R-Baclofen Reverses Social Deficits and Reduces Repetitive Behavior in Two Mouse Models of Autism. Neuropsychopharmacology 40, 2228-2239 (2015).
35. A. Ligsay, A. Van Dijck, D. V. Nguyen, R. Lozano, Y. Chen, E. S. Bickel, D. Hessl, A. Schneider, K. Angkustsiri, F. Tassone, B. Ceulemans, R. F. Kooy, R. J. Hagerman, A randomized double-blind, placebo-controlled trial of ganaxolone in children and adolescents with fragile X syndrome. J Neurodev Disord 9, 26 (2017).
36. E. Berry-Kravis, R. Hagerman, J. Visootsak, D. Budimirovic, W. E. Kaufmann, M. Cherubini, P. Zarevics, K. Walton-Bowen, P. Wang, M. F. Bear, R. L. Carpenter, Arbaclofen in fragile X syndrome: results of phase 3 trials. J Neurodev Disord 9, 3 (2017).
37. Y. Ben-Ari, I. Khalilov, K. T. Kahle, E. Cherubini, The GABA excitatory/inhibitory shift in brain maturation and neurological disorders. Neuroscientist 18, 467-486 (2012).
38. S. Eftekhari, J. Mehvari Habibabadi, M. Najafi Ziarani, S. S. Hashemi Fesharaki, M. Gharakhani, H. Mostafavi, M. T. Joghataei, N. Beladimoghadam, E. Rahimian, M. R. Hadjighassem, Bumetanide reduces seizure frequency in patients with temporal lobe epilepsy. Epilepsia 54, e9-12 (2013).
39. J. S. Soul, A. M. Bergin, C. Stopp, B. Hayes, A. Singh, C. R. Fortuno, D. O'Reilly, K. Krishnamoorthy, F. E. Jensen, V. Rofeberg, M. Dong, A. A. Vinks, D. Wypij, K. J. Staley, G. Boston Bumetanide Trial, A Pilot Randomized, Controlled, Double-Blind Trial of Bumetanide to Treat Neonatal Seizures. Ann Neurol 89, 327-340 (2020).
40. R. Tyzio, R. Nardou, D. C. Ferrari, T. Tsintsadze, A. Shahrokhi, S. Eftekhari, I. Khalilov, V. Tsintsadze, C. Brouchoud, G. Chazal, E. Lemonnier, N. Lozovaya, N. Burnashev, Y. Ben-Ari, Oxytocin-mediated GABA inhibition during delivery attenuates autism pathogenesis in rodent offspring. Science 343, 675-679 (2014).
41. E. Lemonnier, C. Degrez, M. Phelep, R. Tyzio, F. Josse, M. Grandgeorge, N. Hadjikhani, Y. Ben-Ari, A randomised controlled trial of bumetanide in the treatment of autism in children. Transl Psychiatry 2, e202 (2012).
42. E. Fernell, P. Gustafsson, C. Gillberg, Bumetanide for autism: open-label trial in six children. Acta paediatrica 110, 1548-1553 (2020).
43. J. J. Sprengers, D. M. van Andel, N. P. A. Zuithoff, M. G. Keijzer-Veen, A. J. A. Schulp, F. E. Scheepers, M. R. Lilien, B. Oranje, H. Bruining, Bumetanide for Core Symptoms of Autism Spectrum Disorder (BAMBI): A Single Center, Double-Blinded, Participant-Randomized, Placebo-Controlled, Phase-2 Superiority Trial. J Am Acad Child Adolesc Psychiatry S0890-8567, 31290-31299 (2020).
44. J. M. Pearce, Bromide, the first effective antiepileptic agent. Journal of neurology, neurosurgery, and psychiatry 72, 412 (2002).
45. L. Uhr, J. C. Pollard, J. G. Miller, Behavioral effects of chronic administration of psychoactive drugs to anxious patients. Psychopharmacologia 1, 150-168 (1959).
46. A. C. Almeida, F. A. Scorza, A. M. Rodrigues, R. M. Arida, F. N. Carlesso, A. G. Batista, M. A. Duarte, J. C. DaCosta, Combined effect of bumetanide, bromide, and GABAergic agonists: an alternative treatment for intractable seizures. Epilepsy Behav 20, 147-149 (2011).
47. R. C. Woody, Bromide therapy for pediatric seizure disorder intractable to other antiepileptic drugs. J Child Neurol 5, 65-67 (1990).
48. S. Suzuki, K. Kawakami, F. Nakamura, S. Nishimura, K. Yagi, M. Seino, Bromide, in the therapeutic concentration, enhances GABA-activated currents in cultured neurons of rat cerebral cortex. Epilepsy research 19, 89-97 (1994).
49. K. B. Gagnon, N. C. Adragna, R. E. Fyffe, P. K. Lauf, Characterization of glial cell K-Cl cotransport. Cellular physiology and biochemistry : international journal of experimental cellular physiology, biochemistry, and pharmacology 20, 121-130 (2007).
50. R. Kinne, E. Kinne-Saffran, B. Scholermann, H. Schutz, The anion specificity of the sodium-potassium-chloride cotransporter in rabbit kidney outer medulla: studies on medullary plasma membranes. Pflugers Archiv : European journal of physiology 407 Suppl 2, S168-173 (1986).
51. J. A. Becker, D. Clesse, C. Spiegelhalter, Y. Schwab, J. Le Merrer, B. L. Kieffer, Autistic-like syndrome in mu opioid receptor null mice is relieved by facilitated mGluR4 activity. Neuropsychopharmacology 39, 2049-2060 (2014).
52. L. Jamot, H. W. Matthes, F. Simonin, B. L. Kieffer, J. C. Roder, Differential involvement of the mu and kappa opioid receptors in spatial learning. Genes Brain Behav 2, 80-92 (2003).
53. K. M. Jung, M. Sepers, C. M. Henstridge, O. Lassalle, D. Neuhofer, H. Martin, M. Ginger, A. Frick, N. V. DiPatrizio, K. Mackie, I. Katona, D. Piomelli, O. J. Manzoni, Uncoupling of the endocannabinoid signalling complex in a mouse model of fragile X syndrome. Nat Commun 3, 1080 (2012).
54. A. Michalon, M. Sidorov, T. M. Ballard, L. Ozmen, W. Spooren, J. G. Wettstein, G. Jaeschke, M. F. Bear, L. Lindemann, Chronic pharmacological mGlu5 inhibition corrects fragile X in adult mice. Neuron 74, 49-56 (2012).
55. J. Peca, C. Feliciano, J. T. Ting, W. Wang, M. F. Wells, T. N. Venkatraman, C. D. Lascola, Z. Fu, G. Feng, Shank3 mutant mice display autistic-like behaviours and striatal dysfunction. Nature 472, 437-442 (2011).
56. A. S. Tora, X. Rovira, I. Dione, H. O. Bertrand, I. Brabet, Y. De Koninck, N. Doyon, J. P. Pin, F. Acher, C. Goudet, Allosteric modulation of metabotropic glutamate receptors by chloride ions. Faseb J, (2015).
57. B. R. Conklin, Z. Farfel, K. D. Lustig, D. Julius, H. R. Bourne, Substitution of three amino acids switches receptor specificity of Gq alpha to that of Gi alpha. Nature 363, 274-276 (1993).
58. H. W. Matthes, R. Maldonado, F. Simonin, O. Valverde, S. Slowe, I. Kitchen, K. Befort, A. Dierich, M. Le Meur, P. Dolle, E. Tzavara, J. Hanoune, B. P. Roques, B. L. Kieffer, Loss of morphine-induced analgesia, reward effect and withdrawal symptoms in mice lacking the mu-opioid-receptor gene. Nature 383, 819-823 (1996).
59. E. J. Mientjes, I. Nieuwenhuizen, L. Kirkpatrick, T. Zu, M. Hoogeveen-Westerveld, L. Severijnen, M. Rife, R. Willemsen, D. L. Nelson, B. A. Oostra, The generation of a conditional Fmr1 knock out mouse model to study Fmrp function in vivo. Neurobiology of disease 21, 549-555 (2006).
60. J. A. J. Becker, L. P. Pellissier, Y. Corde, T. Laboute, A. Leaute, J. Gandia, J. Le Merrer, Facilitating mGluR4 activity reverses the long-term deleterious consequences of chronic morphine exposure in male mice. Neuropsychopharmacology 46, 1373-1385 (2021).
61. N. Matsuo, K. Tanda, K. Nakanishi, N. Yamasaki, K. Toyama, K. Takao, H. Takeshima, T. Miyakawa, Comprehensive behavioral phenotyping of ryanodine receptor type 3 (RyR3) knockout mice: decreased social contact duration in two social interaction tests. Front Behav Neurosci 3, 3 (2009).
62. Y. Katayama, M. Nishiyama, H. Shoji, Y. Ohkawa, A. Kawamura, T. Sato, M. Suyama, T. Takumi, T. Miyakawa, K. I. Nakayama, CHD8 haploinsufficiency results in autistic-like phenotypes in mice. Nature 537, 675-679 (2016).
63. C. M. Spencer, O. Alekseyenko, E. Serysheva, L. A. Yuva-Paylor, R. Paylor, Altered anxiety-related and social behaviors in the Fmr1 knockout mouse model of fragile X syndrome. Genes Brain Behav 4, 420-430 (2005).
64. J. L. Silverman, M. Yang, C. Lord, J. N. Crawley, Behavioural phenotyping assays for mouse models of autism. Nat Rev Neurosci 11, 490-502 (2010).
65. N. Le Marec, K. Ethier, P. P. Rompre, R. Godbout, Involvement of the medial prefrontal cortex in two alternation tasks using different environments. Brain and cognition 48, 432-436 (2002).
66. A. Moustgaard, J. Hau, N. M. Lind, Effects of dopamine D4 receptor antagonist on spontaneous alternation in rats. Behavioral and brain functions : BBF 4, 49 (2008).
67. D. Delotterie, G. Ruiz, J. Brocard, A. Schweitzer, C. Roucard, Y. Roche, M. F. Suaud-Chagny, K. Bressand, A. Andrieux, Chronic administration of atypical antipsychotics improves behavioral and synaptic defects of STOP null mice. Psychopharmacology (Berl) 208, 131-141 (2010).
68. A. Thomas, A. Burant, N. Bui, D. Graham, L. A. Yuva-Paylor, R. Paylor, Marble burying reflects a repetitive and perseverative behavior more than novelty-induced anxiety. Psychopharmacology (Berl) 204, 361-373 (2009).
69. S. L. Raidal, S. Edwards, Pharmacokinetics of potassium bromide in adult horses. Australian veterinary journal 86, 187-193 (2008).
70. E. G. Weir, A. B. Hastings, The distribution of bromide and chloride in tissues and body fluids. Journal of Biological Chemistry 129, 547-558 (1939).
71. F. X. van Leeuwen, E. M. den Tonkelaar, M. J. van Logten, Toxicity of sodium bromide in rats: effects on endocrine system and reproduction. Food and chemical toxicology : an international journal published for the British Industrial Biological Research Association 21, 383-389 (1983).
72. E. Trinquet, M. Fink, H. Bazin, F. Grillet, F. Maurin, E. Bourrier, H. Ansanay, C. Leroy, A. Michaud, T. Durroux, D. Maurel, F. Malhaire, C. Goudet, J. P. Pin, M. Naval, O. Hernout, F. Chretien, Y. Chapleur, G. Mathis, D-myo-inositol 1-phosphate as a surrogate of D-myo-inositol 1,4,5-tris phosphate to monitor G protein-coupled receptor activation. Analytical biochemistry 358, 126-135 (2006).

## Claims

1. A combination comprising a bromide salt and a Positive Allosteric Modulator (PAM) of mGlu4 receptor for use in the treatment of an Autism Spectrum Disorder (ASD) in a subject in need thereof.

2. The combination for use according to claim 1 to alleviate at least one symptom of ASD in a subject in need thereof, wherein the at least one symptom is social behaviour deficits, stereotypic behaviours and/or excessive anxiety.

3. The combination for use of claim 1 or 2, wherein the bromide salt is sodium bromide.

4. The combination for use of claim 1 or 2, wherein the bromide salt is potassium bromide.

5. The combination for use of claim 1 or 2, wherein the positive allosteric modulator of mGlu4 receptor is VU0155041.

6. A bromide salt in combination with a Positive Allosteric Modulator (PAM) of mGlu4 receptor for use in the treatment of an Autism Spectrum Disorder (ASD) in a subject in need thereof, wherein said bromide salt is administered for enhancing the potency of said positive Allosteric Modulator (PAM) of mGlu4 receptor.

7. The combination for use according to claim 6, wherein the bromide salt is sodium bromide and the Positive Allosteric Modulator (PAM) of mGlu4 receptor is VU0155041.

8. The combination for use of claim 1 to 7, wherein the bromide salt is administrated chronically.

9. The combination for use of claim 1 to 8, wherein the bromide salt is administrated at least 10 mg/kg.

10. A bromide salt and a Positive Allosteric Modulator (PAM) of mGlu4 receptor as a combined preparation for use in the treatment of Autism Spectrum Disorders (ASD), wherein said bromide salt and PAM of mGlu4 are administered simultaneously, separately or sequentially.

11. The combined preparation for use according to claim 10, wherein the bromide salt is sodium bromide and the Positive Allosteric Modulator (PAM) of mGlu4 receptor is VU0155041.

12. A therapeutic composition comprising a bromide salt and a Positive Allosteric Modulator (PAM) of mGlu4 receptor for use in the treatment of Autism Spectrum Disorders (ASD) in a subject in need thereof.

13. The therapeutic composition for use according to claim 12, wherein the bromide salt is sodium bromide and the Positive Allosteric Modulator (PAM) of mGlu4 receptor is VU0155041.

## Patentansprüche

1. Eine Kombination, umfassend ein Bromidsalz und einen Positiven Allosterischen Modulator (PAM) des mGlu4-Rezeptors zur Verwendung bei der Behandlung einer Autismus-Spektrum-Störung (ASS) bei einem Subjekt, das dessen bedarf.

2. Die Kombination zur Verwendung nach Anspruch 1 zur Linderung mindestens eines Symptoms von ASS bei einem Subjekt, das dessen bedarf, wobei das mindestens eine Symptom Defizite im Sozialverhalten, stereotype Verhaltensweisen und/oder übermäßige Angst ist.

3. Die Kombination zur Verwendung nach Anspruch 1 oder 2, wobei das Bromidsalz Natriumbromid ist.

4. Die Kombination zur Verwendung nach Anspruch 1 oder 2, wobei das Bromidsalz Kaliumbromid ist.

5. Die Kombination zur Verwendung nach Anspruch 1 oder 2, wobei der positive allosterische Modulator des mGlu4-Rezeptors VU0155041 ist.

6. Ein Bromidsalz in Kombination mit einem Positiven Allosterischen Modulator (PAM) des mGlu4-Rezeptors zur Verwendung bei der Behandlung einer Autismus-Spektrum-Störung (ASS) bei einem Subjekt, das dessen bedarf, wobei das Bromidsalz zur Steigerung der Wirksamkeit des Positiven Allosterischen Modulators (PAM) des mGlu4-Rezeptors verabreicht wird.

7. Die Kombination zur Verwendung nach Anspruch 6, wobei das Bromidsalz Natriumbromid ist und der Positive Allosterische Modulator (PAM) des mGlu4-Rezeptors VU0155041 ist.

8. Die Kombination zur Verwendung nach den Ansprüchen 1 bis 7, wobei das Bromidsalz chronisch verabreicht wird.

9. Die Kombination zur Verwendung nach den Ansprüchen 1 bis 8, wobei das Bromidsalz mit mindestens 10 mg/kg verabreicht wird.

10. Ein Bromidsalz und ein Positiver Allosterischer Modulator (PAM) des mGlu4-Rezeptors als ein Kombinationspräparat zur Verwendung bei der Behandlung von Autismus-Spektrum-Störungen (ASS), wobei das Bromidsalz und der PAM von mGlu4 gleichzeitig, getrennt oder nacheinander verabreicht werden.

11. Das Kombinationspräparat zur Verwendung nach Anspruch 10, wobei das Bromidsalz Natriumbromid ist und der Positive Allosterische Modulator (PAM) des mGlu4-Rezeptors VU0155041 ist.

12. Eine therapeutische Zusammensetzung, umfassend ein Bromidsalz und einen Positiven Allosterischen Modulator (PAM) des mGlu4-Rezeptors zur Verwendung bei der Behandlung von Autismus-Spektrum-Störungen (ASS) bei einem Subjekt, das dessen bedarf.

13. Die therapeutische Zusammensetzung zur Verwendung nach Anspruch 12, wobei das Bromidsalz Natriumbromid ist und der Positive Allosterische Modulator (PAM) des mGlu4-Rezeptors VU0155041 ist.

## Revendications

1. Une combinaison comprenant un sel de bromure et un Modulateur Allostérique Positif (MAP) du récepteur mGlu4 pour son utilisation dans le traitement d'un Trouble du Spectre de l'Autisme (TSA) chez un sujet en ayant besoin.

2. La combinaison pour son utilisation selon la revendication 1 pour soulager au moins un symptôme de TSA chez un sujet en ayant besoin, dans laquelle le au moins un symptôme est des déficits de comportement social, des comportements stéréotypés et/ou une anxiété excessive.

3. La combinaison pour son utilisation selon la revendication 1 ou 2, dans laquelle le sel de bromure est le bromure de sodium.

4. La combinaison pour son utilisation selon la revendication 1 ou 2, dans laquelle le sel de bromure est le bromure de potassium.

5. La combinaison pour son utilisation selon la revendication 1 ou 2, dans laquelle le modulateur allostérique positif du récepteur mGlu4 est le VU0155041.

6. Un sel de bromure en combinaison avec un Modulateur Allostérique Positif (MAP) du récepteur mGlu4 pour son utilisation dans le traitement d'un Trouble du Spectre de l'Autisme (TSA) chez un sujet en ayant besoin, dans lequel ledit sel de bromure est administré pour augmenter la puissance dudit Modulateur Allostérique Positif (MAP) du récepteur mGlu4.

7. La combinaison pour son utilisation selon la revendication 6, dans laquelle le sel de bromure est le bromure de sodium et le Modulateur Allostérique Positif (MAP) du récepteur mGlu4 est le VU0155041.

8. La combinaison pour son utilisation selon les revendications 1 à 7, dans laquelle le sel de bromure est administré de manière chronique.

9. La combinaison pour son utilisation selon les revendications 1 à 8, dans laquelle le sel de bromure est administré à au moins 10 mg/kg.

10. Un sel de bromure et un Modulateur Allostérique Positif (MAP) du récepteur mGlu4 en tant que préparation combinée pour son utilisation dans le traitement des Troubles du Spectre de l'Autisme (TSA), dans laquelle ledit sel de bromure et ledit MAP de mGlu4 sont administrés simultanément, séparément ou séquentiellement.

11. La préparation combinée pour son utilisation selon la revendication 10, dans laquelle le sel de bromure est le bromure de sodium et le Modulateur Allostérique Positif (MAP) du récepteur mGlu4 est le VU0155041.

12. Une composition thérapeutique comprenant un sel de bromure et un Modulateur Allostérique Positif (MAP) du récepteur mGlu4 pour son utilisation dans le traitement des Troubles du Spectre de l'Autisme (TSA) chez un sujet en ayant besoin.

13. La composition thérapeutique pour son utilisation selon la revendication 12, dans laquelle le sel de bromure est le bromure de sodium et le Modulateur Allostérique Positif (MAP) du récepteur mGlu4 est le VU0155041.
